# EUROPEAN PATENT SPECIFICATION

(11) **EP 1 196 440 B1**
(45) Date of publication and mention of the grant of the patent: **03.03.2004**
(21) Application number: 00943110.7
(22) Date of filing: 23.06.2000
(51) Int. Cl.: C07K 14/47, A61K 38/17, A61P 9/00

(54) **THERAPEUTIC PEPTIDES DERIVED FROM SUBSEQUENCES OF BPI**
THERAPEUTISCHE PEPTIDE AUS UNTERSEQUENZEN VON BPI
PEPTIDIQUES THERAPEUTIQUES DERIVES DE SUBSEQUENCES DE BPI

(30) Priority: 25.06.1999 US 344219; 25.06.1999 US 344827
(43) Date of publication of application: 17.04.2002
(73) Proprietor: XOMA Technology Ltd., Berkeley, CA 94710 (US)
(72) Inventor: LITTLE, Roger, G., Benicia, CA 94510 (US); LIN, Jong-Jye, Hercules, CA 94547 (US); GIKONYO, J., G., Kinyua, Berkeley, CA 94708 (US)
(74) Representative: Richardson, Kate
(86) International application number: PCT/US2000/017358
(87) International publication number: WO 2001/000655

(56) References cited:
- WO-A-94/20532
- WO-A-95/05393
- WO-A-99/06440

### SEQUENCE LISTING

<110> XOMA Technology Ltd. c/o XOMA (US) LLC
   Little, II, Roger G.
   Lin, Jong J.
   Gikonyo, J. G. Kinyua
<120> Therapeutic Peptide - Based Constructs
<130> 11044WO01
   <140>
   <141>
<150> 09/344,219
   <151> 1999-06-25
<150> 09/344,827
   <151> 1999-06-25
<160> 28
<170> PatentIn Ver. 2.1
<210> 1
   <211> 15
   <212> PRT
   <213> Homo sapiens
<220>
   <223> XMP.394
<220>
   <221> SITE
   <222> (5)
   <223> /label=Substituted-Ala note=position 5 is 1-naph-ala
<220>
   <221> SITE
   <222> (9)
   <223> /label=Substituted-Ala note=position 9 is 1-naph-ala
<220>
   <221> SITE
   <222> (1) .. (15)
   <223> /Label=D Amino Acids/note=Positions 1-15 are D-amino acids
<220>
   <221> SITE
   <222> (15)
   <223> AMIDATION /label=Amidation note=The C-terminus is Amidated
<210> 2
   <211> 14
   <212> PRT
   <213> Homo sapiens
<220>
   <223> XMP.624
<220>
   <221> SITE
   <222> (5)
   <223> /label=substituted-Ala note=position 5 is 1-naph-ala
<220>
   <221> SITE
   <222> (9)
   <223> /label=substituted-Ala note=position 9 is 1-naph-ala
<220>
   <221> SITE
   <222> (1)..(14)
   <223> /Label=D Amino Acids/note=Positions 1-14 are D-amino acids
<220>
   <221> SITE
   <222> (14)
   <223> AMIDATION /label=Amidation note=The C=terminus is Amidated
<400> 2
   Lys Leu Phe Arg Ala Gln Ala Lys Ala Lys Gly Ser Ile Lys
    1               5                  10
<210> 3
   <211> 13
   <212> PRT
   <213> Homo sapiens
<220>
   <223> XMP.625
<220>
   <221> SITE
   <222> (5)
   <223> /label=Substituted-Ala note=position 5 is 1-naph-ala
<220>
   <221> SITE
   <222> (9)
   <223> /label=substituted-Ala note=position 9 is 1-naph-ala
<220>
   <221> SITE
   <222> (1)..(13)
   <223> /Label=D Amino Acid/note=Positions 1-13 are D-amino acids
<220>
   <221> SITE
   <222> (13)
   <223> AMIDATION /label-=Amidation note=The C-terminus is Amidated
<400> 3
   Lys Leu Phe Arg Ala Gln Ala Lys Ala Lys Gly Ser Ile
   1 5 10
<210> 4
   <211> 12
   <212> PRT
   <213> Homo sapiens
<220>
   <223> XMP.626
<220>
   <221> SITE
   <222> (5)
   <223> /label-Substituted-Ala note=position 5 is 1-naph-ala
<220>
   <221> SITE
   <222> (9)
   <223> /label-Substituted-Ala note=position 9 is 1-naph-ala
<220>
   <221> SITE
   <222> (1)..(12)
   <223> /Label=D Amino Acids/note=Positions 1-12 are D-amino acids
<220>
   <221> SITE
   <222> (12)
   <223> AMIDATION /label=Amidation note=The C-terminus is Amidated
<400> 4
   Lys Leu Phe Arg Ala Gln Ala Lys Ala Lys Gly Ser
   1 5 10
<210> 5
   <211> 11
   <212> PRT
   <213> Homo sapiens
<220>
   <223> XMP.627
<220>
   <221> SITE
   <222> (5)
   <223> /label=Substituted Ala note=position 5 is 1-naph-ala
<220>
   <221> SITE
   <222> (9)
   <223> /label=Substituted Ala note=position 9 is 1-naph-ala
<220>
   <221> SITE
   <222> (1) .. (11)
   <223> /Label=D Amino Acids/note=Positions 1-11 are D-amino acids
<220>
   <221> SITE
   <222> (11)
   <223> AMIDATION /label=Amidation note=The C-terminus is Amidated
<400> 5
   Lys Leu Phe Arg Ala Gln Ala Lys Ala Lys Gly
    1               5                   10
<210> 6
   <211> 10
   <212> PRT
   <213> Homo sapiens
<220>
   <223> XMP.628
<220>
   <221> SITE
   <222> (5)
   <223> /label=Substituted-Ala note=position 5 is 1-naph-ala
<220>
   <221> SITE
   <222> (6)
   <223> /label-Substituted-Ala note=position 9 is 1-naph-ala
<220>
   <221> SITE
   <222> (1)..(10)
   <223> /Label=D Amino Acid/note=Positions 1-10 are D-amino acids
<220>
   <221> SITE
   <222> (10)
   <223> AMIDATION /label=Amidation note=The C-terminus is Amidated
<400> 6
   Lys Leu Phe Arg Ala Gln Ala Lys Ala Lys
    1               5                   10
<210> 7
   <211> 9
   <212> PRT
   <213> Homo sapiens
<220>
   <223> XMP.629
<220>
   <221> SITE
   <222> (5)
   <223> /label=Substituted-Ala note=position 5 is 1-naph-ala
<220>
   <221> SITE
   <222> (9)
   <223> /label=Substituted-Ala note=position 9 is 1-naph-ala
<220>
   <221> SITE
   <222> (1)..(9)
   <223> /Label=D Amino Acids/note=Positions 1-9 are D-amino acids
<220>
   <221> SITE
   <222> (9)
   <223> AMIDATION /label=Amidation note=The C-terminus is Amidated
<400> 7
   Lys Leu Phe Arg Ala Gln Ala Lys Ala
   1               5
<210> 8
   <211> 8
   <212> PRT
   <213> Homo sapiens
<220>
   <223> XMP.630
<220>
   <221> SITE
   <222> (5)
   <223> /label=Substituted-Ala note=position 5 is 1-naph-ala
<220>
   <221> SITE
   <222> (1)..(8)
   <223> /Label=D Amino Acids/note=Positions 1-8 are D-amino acids
<220>
   <221> SITE
   <222> (8)
   <223> AMIDATION /label=Amidation note=The C-terminus is Amidated
<400> 8
   Lys Leu Phe Arg Ala Gln Ala Lys
   1               5
<210> 9
   <211> 14
   <212> PRT
   <213> Homo sapiens
<220>
   <223> XMP.656
<220>
   <221> SITE
   <222> (5)
   <223> /label=Substituted-Ala note=position 5 is 1-naph-ala
<220>
   <221> SITE
   <222> (9)
   <223> /label=Substituted-Ala note=position 9 is 1-naph-ala
<220>
   <221> SITE
   <222> (1)..(14)
   <223> /Label=D Amino Acids/note=Positions 1-14 are D-amino acids
<220>
   <221> SITE
   <222> (14)
   <223> AMIDATION /label=Amidation note=The C-terminus is Amidated
<400> 9
   Lys Leu Phe Arg Ala Gln Ala Lys Ala Lys Gly Ile Lys Ile
    1               5                   10
<210> 10
   <211> 11
   <212> PRT
   <213> Homo sapiens
<220>
   <223> XMP.679
<220>
   <221> SITE
   <222> (5)
   <223> /label=Substituted-Ala note=position 5 is 1-naph-ala
<220>
   <221> SITE
   <222> (9)
   <223> /label=Substituted-Ala note=position 9 is 1-naph-ala
<220>
   <221> SITE
   <222> (1)..(11)
   <223> /Label=D Amino Acids/note=Positions 1-11 are D-Amino Acids
<220>
   <221> SITE
   <222> (11)
   <223> AMIDATION /label=Amidation note=The C-terminus is Amidated
<400> 10
   Lys Leu Phe Lys Ala Gln Ala Lys Ala Lys Gly
   1 5 10
<210> 11
   <211> 9
   <212> PRT
   <213> Homo sapiens
<220>
   <223> XMP.684
<220>
   <221> SITE
   <222> (1)
   <223> /label=Substituted Ala note=position 1 is 4-biphenyl-Ala
<220>
   <221> SITE
   <222> (6)
   <223> /label=Substituted Ala note=position 6 is 1-naph-ala
<220>
   <221> SITE
   <222> (1)
   <223> Position 1 is an L-amino acid
<220>
   <221> SITE
   <222> (2)..(9)
   <223> /Label-D Amino Acids/note=Positions 2-9 are D-amino acids
<220>
   <221> SITE
   <222> (9)
   <223> AMIDATION /label=Amidation note=The C-terminus is Amidated
<400> 11
   Ala Lys Leu Phe Arg Ala Gln Ala Lys
   1 5
<210> 12
   <211> 8
   <212> PRT
   <213> Homo sapiens
<220>
   <223> XMP.685
<220>
   <221> SITE
   <222> (1)..(8)
   <223> Positions 1-4 and 6-8 are D-amino acids
<220>
   <221> SITE
   <222> (5)
   <223> Position 5 is an L-amino acid
<220>
   <221> SITE
   <222> (5)
   <223> Position 5 is substituted with 4-biphenyl-Ala
<220>
   <221> SITE
   <222> (8)
   <223> AMIDATION=The C-terminus is Amidated
<400> 12
   Lys Leu Phe Arg Ala Gln Ala Lys
    1               5
<210> 13
   <211> 8
   <212> PRT
   <213> Homo sapiens
<220>
   <223> XMP.725
<220>
   <221> SITE
   <222> (1)..(8)
   <223> Positions 1-8 are D-amino acids
<220>
   <221> SITE
   <222> (5)
   <223> Position 5 is substituted with 4-biphenyl-ala
<220>
   <221> SITE
   <222> (8)
   <223> AMIDATION=The C-terminus is Amidated
<400> 13
   Lys Leu Phe Arg Ala Gln Ala Lys
    1               5
<210> 14
   <211> 11
   <212> PRT
   <213> Homo sapiens
<220>
   <223> XMP.728
<220>
   <221> SITE
   <222> (1)..(11)
   <223> Positions 1-11 are D-amino acids
<220>
   <221> SITE
   <222> (5)
   <223> Position 5 is substituted with 4-biphenyl-ala
<220>
   <221> SITE
   <222> (9)
   <223> Position 9 is substituted with 4-biphenyl-ala
<220>
   <221> SITE
   <222> (11)
   <223> AMIDATION=The C-terminus is Amidated
<400> 14
   Lys Leu Phe Lys Ala Gln Ala Lys Ala Lys Gly
    1               5                   10
<210> 15
   <211> 9
   <212> PRT
   <213> Homo sapiens
<220>
   <223> XMP.760
<220>
   <221> SITE
   <222> (1)..(9)
   <223> Positions 1-9 are D-amino acids
<220>
   <221> SITE
   <222> (5)
   <223> Position 5 is substituted with 1-naph-ala
<220>
   <221> SITE
   <222> (9)
   <223> Position 9 is substituted with 1-naph-ala
<220>
   <221> SITE
   <222> (9)
   <223> AMIDATION=The C-terminus is Amidated
<400> 15
   Lys Ala Phe Arg Ala Gln Ala Lys Ala
   1 5
<210> 16
   <211> 11
   <212> PRT
   <213> Homo sapiens
<220>
   <223> XMP.764
<220>
   <221> SITE
   <222> (1)..(11)
   <223> Positions 1-11 are D-amino acids
<220>
   <221> SITE
   <222> (5)
   <223> Position 5 is substituted with 1-naph-ala
<220>
   <221> SITE
   <222> (9)
   <223> Position 9 is substituted with 1-naph-ala
<220>
   <221> SITE
   <222> (11)
   <223> AMIDATION=The C-terminus is Amidated
<400> 16
   Lys Ala Phe Lys Ala Gln Ala Lys Ala Lys Gly
   1 5 10
<210> 17
   <211> 9
   <212> PRT
   <213> Homo sapiens
<220>
   <223> XMP.776
<220>
   <221> SITE
   <222> (1)..(9)
   <223> Positions 1-9 are D-amino acids
<220>
   <221> SITE
   <222> (5)
   <223> Position 5 is substituted with 1-naph-ala
<220>
   <221> SITE
   <222> (9)
   <223> Position 9 is substituted with 1-naph-ala
<220>
   <221> SITE
   <222> (9)
   <223> AMIDATION=The C-terminus is Amidated
<400> 17
   Lys Leu Phe Lys Ala Gln Ala Lys Ala
    1               5
<210> 18
   <211> 9
   <212> PRT
   <213> Homo sapiens
<220>
   <223> XMP.778
<220>
   <221> SITE
   <222> (1)..(9)
   <223> Positions 1-9 are D-amino acids
<220>
   <221> SITE
   <222> (2)
   <223> Position 2 is aminoisobutyric acid
<220>
   <221> SITE
   <222> (5)
   <223> Position 5 is substituted with 1-naph-ala
<220>
   <221> SITE
   <222> (9)
   <223> Position 9 is substituted with 1-naph-ala
<220>
   <221> SITE
   <222> (9)
   <223> AMIDATION=The C-terminus is Amidated
<400> 18
   Lys Xaa Phe Arg Ala Gln Ala Lys Ala
    1               5
<210> 19
   <211> 8
   <212> PRT
   <213> Homo sapiens
<220>
   <223> XMP.661
<220>
   <221> SITE
   <222> (1)..(8)
   <223> Positions 1-8 are D-amino acids
<220>
   <221> SITE
   <222> (5)
   <223> Position 5 is substituted with 1-naph-ala
<220>
   <221> SITE
   <222> (1)
   <223> Position 1 is derivatized at the alpha-amino group with 2-biphenyl carbonyl
<220>
   <221> SITE
   <222> (8)
   <223> AMIDATION=The C-terminus is Amidated
<400> 19
   Lys Leu Phe Arg Ala Gln Ala Lys
    1               5
<210> 20
   <211> 8
   <212> PRT
   <213> Homo sapiens
<220>
   <223> XMP.664
<220>
   <221> SITE
   <222> (1)..(8)
   <223> Positions 1-8 are D-amino acids
<220>
   <221> SITE
   <222> (5)
   <223> Position 5 is substituted with 1- naph-ala
<220>
   <221> SITE
   <222> (1)
   <223> Position 1 is derivatized at the alpha-amino group with 4-biphenyl carbonyl
<220>
   <221> SITE
   <222> (8)
   <223> AMIDATION=The C-terminus is Amidated
<400> 20 <210> 21
   <211> 8
   <212> PRT
   <213> Homo sapiens
<220>
   <223> XMP.666
<220>
   <221> SITE
   <222> (1)..(8)
   <223> Positions 1-8 are D-amino acids
<220>
   <221> SITE
   <222> (5)
   <223> Position 5 is substituted with 1-naph-ala
<220>
   <221> SITE
   <222> (1)
   <223> Position 1 is derivatized at the alpha-amino group with 2-(2-naphthyl) acetyl
<220>
   <221> SITE
   <222> (8)
   <223> AMIDATION=The C-terminus is Amidated
<400> 21
<210> 22
   <211> 8
   <212> PRT
   <213> Homo sapiens
<220>
   <223> XMP.671
<220>
   <221> SITE
   <222> (1)..(8)
   <223> Positions 1-8 are D-amino acids
<220>
   <221> SITE
   <222> (5)
   <223> Position 5 is substituted with 1-naph-ala
<220>
   <221> SITE
   <222> (1)
   <223> Position 1 is derivatized at the alpha-amino group with 2-(1-naphthyl) acetyl
<220>
   <221> SITE
   <222> (8)
   <223> AMIDATION=The C-terminus is Amidated
<400> 22
   Lys Leu Phe Arg Ala Gln Ala Lys
    1               5
<210> 23
   <211> 8
   <212> PRT
   <213> Homo sapiens
<220>
   <223> XMP.699
<220>
   <221> SITE
   <222> (1)
   <223> Positions 1-8 are D-amino acids
<220>
   <221> SITE
   <222> (5)
   <223> Position 5 is substituted with 1-naph-ala
<220>
   <221> SITE
   <222> (1)
   <223> Position 1 is derivatized at the alpha-amino group with 2-biphenylene carbonyl
<220>
   <221> SITE
   <222> (8)
   <223> AMIDATION=The C-terminus is Amidated
<400> 23
   Lys Leu Phe Arg Ala Gln Ala Lys
    1               5
<210> 24
   <211> 8
   <212> PRT
   <213> Homo sapiens
<220>
   <223> XMP.767
<220>
   <221> SITE
   <222> (1)..(8)
   <223> Positions 1-8 are D-amino acids
<220>
   <221> SITE
   <222> (5)
   <223> Position 5 is substituted with 1-naph-ala
<220>
   <221> SITE
   <222> (1)
   <223> Position 1 is derivatized at the alpha-amino group with 4-biphenyl carbonyl
<220>
   <221> SITE
   <222> (8)
   <223> AMIDATION=The C-terminus is Amidated
<400> 24
   Lys Leu Phe Lys Ala Gln Ala Lys
   1               5
<210> 25
   <211> 8
   <212> PRT
   <213> Homo sapiens
<220>
   <223> XMP.768
<220>
   <221> SITE
   <222> (1)..(8)
   <223> Positions 1-8 are D-amino acids
<220>
   <221> SITE
   <222> (5)
   <223> Position 5 is substituted with 4-biphenyl-ala
<220>
   <221> SITE
   <222> (1)
   <223> Position 1 is derivatized at the alpha-amino group with 4-biphenyl carbonyl
<220>
   <221> SITE
   <222> (8)
   <223> AMIDATION=The C-terminus is Amidated
<400> 25
   Lys Leu Phe Arg Ala Gln Ala Lys
   1               5
<210> 26
   <211> 8
   <212> PRT
   <213> Homo sapiens
<220>
   <223> XMP.769
<220>
   <221> SITE
   <222> (1)..(8)
   <223> Positions 1-8 are D-amino acids
<220>
   <221> SITE
   <222> (5)
   <223> Position 5 is substituted with 4-biphenyl-ala
<220>
   <221> SITE
   <222> (1)
   <223> Position 1 is derivatized at the alpha-amino group with 4-biphenyl carbonyl
<220>
   <221> SITE
   <222> (8)
   <223> AMIDATION=The C-terminus is Amidated
<400> 26
   Lys Leu Phe Lys Ala Gln Ala Lys
   1               5
<210> 27
   <211> 1813
   <212> DNA
   <213> Homo sapiens
<220>
   <221> CDS
   <222> (31)..(1491)
<220>
   <221> mat_peptide
   <222> (124)..(1491)
<220>
   <223> rBPI
<400> 27
   caggccttga ggttttggca gctctggagg atg aga gag aac atg gcc agg ggc  54
                                    Met Arg Glu Asn Met Ala Arg Gly
                                            -30                 -25
   cct tgc aac gcg ccg aga tgg gtg tcc ctg atg gtg ctc gtc gcc ata   102
   Pro Cys Asn Ala Pro Arg Trp Val Ser Leu Met Val Leu Val Ala Ile
               -20                 -15                 -10
   ggc acc gcc gtg aca gcg gcc gtc aac cct ggc gtc gtg gtc agg atc   150
   Gly Thr Ala Val Thr Ala Ala Val Asn Pro Gly Val Val Val Arg Ile
           -5                  -1   1               5
   tcc cag aag ggc ctg gac tac gcc agc cag cag ggg acg gcc gct ctg   198
   Ser Gln Lys Gly Leu Asp Tyr Ala Ser Gln Gln Gly Thr Ala Ala Leu
    10                  15                  20                  25
   cag aag gag ctg aag agg atc aag att cct gac tac tca gac agc ttt   246
   Gln Lys Glu Leu Lys Arg Ile Lys Ile Pro Asp Tyr Ser Asp Ser Phe
                    30                  35                  40
   aag atc aag cat ctt ggg aag ggg cat tat agc ttc tac agc atg gac   294
   Lys Ile Lys His Leu Gly Lys Gly His Tyr Ser Phe Tyr Ser Met Asp
                45                  50                  55
   atc cgt gaa ttc cag ctt ccc agt tcc cag ata agc atg gtg ccc aat   342
   Ile Arg Glu Phe Gln Leu Pro Ser Ser Gln Ile Ser Met Val Pro Asn
            60                  65                  70
   gtg ggc ctt aag ttc tcc atc agc aac gcc aat atc aag atc agc ggg   390
   Val Gly Leu Lys Phe Ser Ile Ser Asn Ala Asn Ile Lys Ile Ser Gly
        75                  80                  85
   aaa tgg aag gca caa aag aga ttc tta aaa atg agc ggc aat ttt gac    438
   Lys Trp Lys Ala Gln Lys Arg Phe Leu Lys Met Ser Gly Asn Phe Asp
    90                  95                 100                 105
   ctg agc ata gaa ggc atg tcc att tcg gct gat ctg aag ctg ggc agt    486
   Leu Ser Ile Glu Gly Met Ser Ile Ser Ala Asp Leu Lys Leu Gly Ser
                   110                 115                 120
   aac ccc acg tca ggc aag ccc acc atc acc tgc tcc agc tgc agc agc    534
   Asn Pro Thr Ser Gly Lys Pro Thr Ile Thr Cys Ser Ser Cys Ser Ser
               125                 130                 135
   cac atc aac agt gtc cac gtg cac atc tca aag agc aaa gtc ggg tgg    582
   His Ile Asn Ser Val His Val His Ile Ser Lys Ser Lys Val Gly Trp
           140                 145                 150
   ctg atc caa ctc ttc cac aaa aaa att gag tct gcg ctt cga aac aag    630
   Leu Ile Gln Leu Phe His Lys Lys Ile Glu Ser Ala Leu Arg Asn Lys
       155                 160                 165
   atg aac agc cag gtc tgc gag aaa gtg acc aat tct gta tcc tcc aag    678
   Met Asn Ser Gln Val Cys Glu Lys Val Thr Asn Ser Val Ser Ser Lys
   170                 175                 180                 185
   ctg caa cct tat ttc cag act ctg cca gta atg acc aaa ata gat tct    726
   Leu Gln Pro Tyr Phe Gln Thr Leu Pro Val Met Thr Lys Ile Asp Ser
                   190                 195                 200
   gtg gct gga atc aac tat ggt ctg gtg gca cct cca gca acc acg gct    774
   Val Ala Gly Ile Asn Tyr Gly Leu Val Ala Pro Pro Ala Thr Thr Ala
               205                 210                 215
   gag acc ctg gat gta cag atg aag ggg gag ttt tac agt gag aac cac    822
   Glu Thr Leu Asp Val Gln Met Lys Gly Glu Phe Tyr Ser Glu Asn His
           220                 225                 230
   cac aat cca cct ccc ttt gct cca cca gtg atg gag ttt ccc gct gcc    870
   His Asn Pro Pro Pro Phe Ala Pro Pro Val Met Glu Phe Pro Ala Ala
       235                 240                 245
   cat gac cgc atg gta tac ctg ggc ctc tca gac tac ttc ttc aac aca    918
   His Asp Arg Met Val Tyr Leu Gly Leu Ser Asp Tyr Phe Phe Asn Thr
   250                 255                 260                 265
   gcc ggg ctt gta tac caa gag gct ggg gtc ttg aag atg acc ctt aga    966
   Ala Gly Leu Val Tyr Gln Glu Ala Gly Val Leu Lys Met Thr Leu Arg
                   270                 275                 280
   gat gac atg att cca aag gag tcc aaa ttt cga ctg aca acc aag ttc    1014
   Asp Asp Met Ile Pro Lys Glu Ser Lys Phe Arg Leu Thr Thr Lys Phe
               285                 290                 295
   ttt gga acc ttc cta cct gag gtg gcc aag aag ttt ccc aac atg aag    1062
   Phe Gly Thr Phe Leu Pro Glu Val Ala Lys Lys Phe Pro Asn Met Lys
           300                 305                 310
   ata cag atc cat gtc tca gcc tcc acc ccg cca cac ctg tct gtg cag   1110
   Ile Gln Ile His Val Ser Ala Ser Thr Pro Pro His Leu Ser Val Gln
       315                 320                 325
   ccc acc ggc ctt acc ttc tac cct gcc gtg gat gtc cag gcc ttt gcc   1158
   Pro Thr Gly Leu Thr Phe Tyr Pro Ala Val Asp Val Gln Ala Phe Ala
   330                 335                 340                 345
   gtc ctc ccc aac tcc tcc ctg gct tcc ctc ttc ctg att ggc atg cac   1206
   Val Leu Pro Asn Ser Ser Leu Ala Ser Leu Phe Leu Ile Gly Met His
                   350                 355                 360
   aca act ggt tcc atg gag gtc agc gcc gag tcc aac agg ctt gtt gga   1254
   Thr Thr Gly Ser Met Glu Val Ser Ala Glu Ser Asn Arg Leu Val Gly
               365                 370                 375
   gag ctc aag ctg gat agg ctg ctc ctg gaa ctg aag cac tca aat att   1302
   Glu Leu Lys Leu Asp Arg Leu Leu Leu Glu Leu Lys His Ser Asn Ile
           380                 385                 390
   ggc ccc ttc ccg gtt gaa ttg ctg cag gat atc atg aac tac att gta   1350
   Gly Pro Phe Pro Val Glu Leu Leu Gln Asp Ile Met Asn Tyr Ile Val
       395                 400                 405
   ccc att ctt gtg ctg ccc agg gtt aac gag aaa cta cag aaa ggc ttc   1398
   Pro Ile Leu Val Leu Pro Arg Val Asn Glu Lys Leu Gln Lys Gly Phe
   410                 415                 420                 425
   cct ctc ccg acg ccg gcc aga gtc cag ctc tac aac gta gtg ctt cag   1446
   Pro Leu Pro Thr Pro Ala Arg Val Gln Leu Tyr Asn Val Val Leu Gln
                   430                 435                 440
   cct cac cag aac ttc ctg ctg ttc ggt gca gac gtt gtc tat aaa       1491
   Pro His Gln Asn Phe Leu Leu Phe Gly Ala Asp Val Val Tyr Lys
               445                 450                 455
   tgaaggcacc aggggtgccg ggggctgtca gccgcacctg ttcctgatgg gctgtggggc 1551
   accggctgcc tttccccagg gaatcctctc cagatcttaa ccaagagccc cttgcaaact 1611
   tcttcgactc agattcagaa atgatctaaa cacgaggaaa cattattcat tggaaaagtg 1671
   catggtgtgt attttaggga ttatgagctt ctttcaaggg ctaaggctgc agagatattt 1731
   cctccaggaa tcgtgtttca attgtaacca agaaatttcc atttgtgctt catgaaaaaa 1791
   aacttctggt ttttttcatg tg                                          1813
<210> 28
   <211> 487
   <212> PRT
   <213> Homo sapiens
   <223> rBPI
<400> 28
   Met Arg Glu Asn Met Ala Arg Gly Pro Cys Asn Ala Pro Arg Trp Val
       -30                 -25                 -20
   Ser Leu Met Val Leu Val Ala Ile Gly Thr Ala Val Thr Ala Ala Val
   -15                 -10                 -5                  -1   1
   Asn Pro Gly Val Val Val Arg Ile Ser Gln Lys Gly Leu Asp Tyr Ala
               5                   10                  15
   Ser Gln Gln Gly Thr Ala Ala Leu Gln Lys Glu Leu Lys Arg Ile Lys
           20                  25                  30
   Ile Pro Asp Tyr Ser Asp Ser Phe Lys Ile Lys His Leu Gly Lys Gly
       35                  40                  45
   His Tyr Ser Phe Tyr Ser Met Asp Ile Arg Glu Phe Gln Leu Pro Ser
   50                  55                  60                  65
   Ser Gln Ile Ser Met Val Pro Asn Val Gly Leu Lys Phe Ser Ile Ser
                   70                  75                  80
   Asn Ala Asn Ile Lys Ile Ser Gly Lys Trp Lys Ala Gln Lys Arg Phe
               85                  90                  95
   Leu Lys Met Ser Gly Asn Phe Asp Leu Ser Ile Glu Gly Met Ser Ile
           100                 105                 110
   Ser Ala Asp Leu Lys Leu Gly Ser Asn Pro Thr Ser Gly Lys Pro Thr
       115                 120                 125
   Ile Thr Cys Ser Ser Cys Ser Ser His Ile Asn Ser Val His Val His
   130                 135                 140                 145
   Ile Ser Lys Ser Lys Val Gly Trp Leu Ile Gln Leu Phe His Lys Lys
                   150                 155                 160
   Ile Glu Ser Ala Leu Arg Asn Lys Met Asn Ser Gln Val Cys Glu Lys
               165                 170                 175
   Val Thr Asn Ser Val Ser Ser Lys Leu Gln Pro Tyr Phe Gln Thr Leu
           180                 185                 190
   Pro Val Met Thr Lys Ile Asp Ser Val Ala Gly Ile Asn Tyr Gly Leu
       195                 200                 205
   Val Ala Pro Pro Ala Thr Thr Ala Glu Thr Leu Asp Val Gln Met Lys
   210                 215                 220                 225
   Gly Glu Phe Tyr Ser Glu Asn His His Asn Pro Pro Pro Phe Ala Pro
                   230                 235                 240
   Pro Val Met Glu Phe Pro Ala Ala His Asp Arg Met Val Tyr Leu Gly
               245                 250                 255
   Leu Ser Asp Tyr Phe Phe Asn Thr Ala Gly Leu Val Tyr Gln Glu Ala
           260                 265                 270
   Gly Val Leu Lys Met Thr Leu Arg Asp Asp Met Ile Pro Lys Glu Ser
       275                 280                 285
   Lys Phe Arg Leu Thr Thr Lys Phe Phe Gly Thr Phe Leu Pro Glu Val
   290                 295                 300                 305
   Ala Lys Lys Phe Pro Asn Met Lys Ile Gln Ile His Val Ser Ala Ser
                   310                 315                 320
   Thr Pro Pro His Leu Ser Val Gln Pro Thr Gly Leu Thr Phe Tyr Pro
               325                 330                 335
   Ala Val Asp Val Gln Ala Phe Ala Val Leu Pro Asn Ser Ser Leu Ala
           340                 345                 350
   Ser Leu Phe Leu Ile Gly Met His Thr Thr Gly Ser Met Glu Val Ser
       355                 360                 365
   Ala Glu Ser Asn Arg Leu Val Gly Glu Leu Lys Leu Asp Arg Leu Leu
   370                 375                 380                 385
   Leu Glu Leu Lys His Ser Asn Ile Gly Pro Phe Pro Val Glu Leu Leu
                   390                 395                 400
   Gln Asp Ile Met Asn Tyr Ile Val Pro Ile Leu Val Leu Pro Arg Val
               405                 410                 415
   Asn Glu Lys Leu Gln Lys Gly Phe Pro Leu Pro Thr Pro Ala Arg Val
           420                 425                 430
   Gln Leu Tyr Asn Val Val Leu Gln Pro His Gln Asn Phe Leu Leu Phe
       435                 440                 445
   Gly Ala Asp Val Val Tyr Lys
   450                 455

## Description

### FIELD OF THE INVENTION

The present invention relates generally to small peptide-based constructs that have 8 to 15 amino acid moieties, including derivatized constructs. The sequences of these constructs are designed and prepared based on a reverse subsequence (99-85) of amino acids identified and selected from Domain II of bactericidal/ permeability-increasing protein (BPI). The invention further relates to therapeutic uses of such constructs due to their heparin-related properties of heparin binding, heparin neutralization, inhibition of endothelial cell proliferation and/or inhibition of angiogenesis, *e.g.,* inhibition of *in vivo* neovascularization, including in models of chronic inflammatory disease states and metastatic tumors.

### BACKGROUND OF THE INVENTION

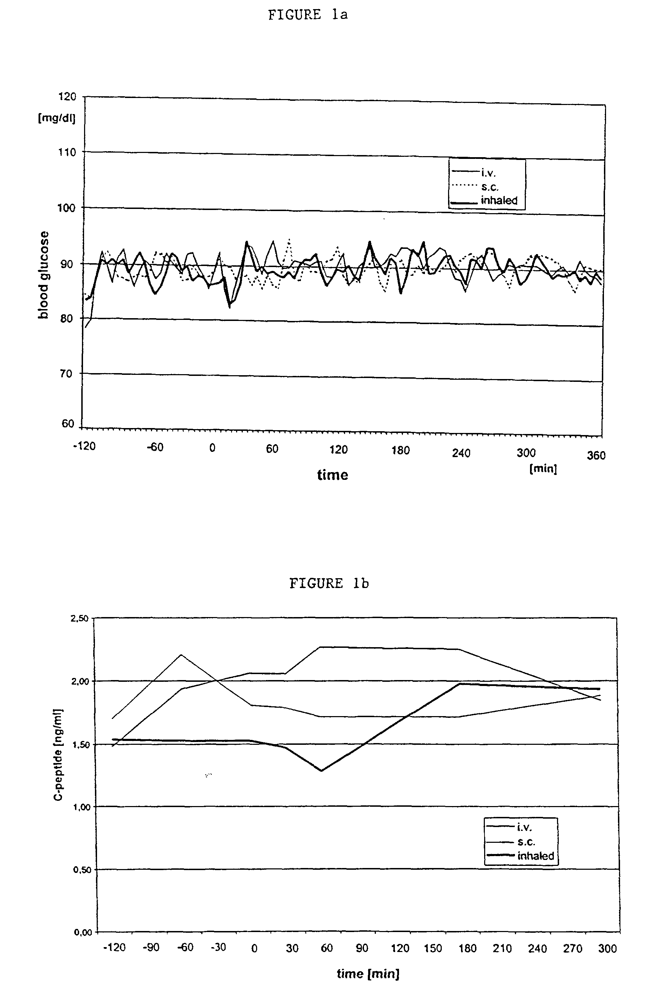

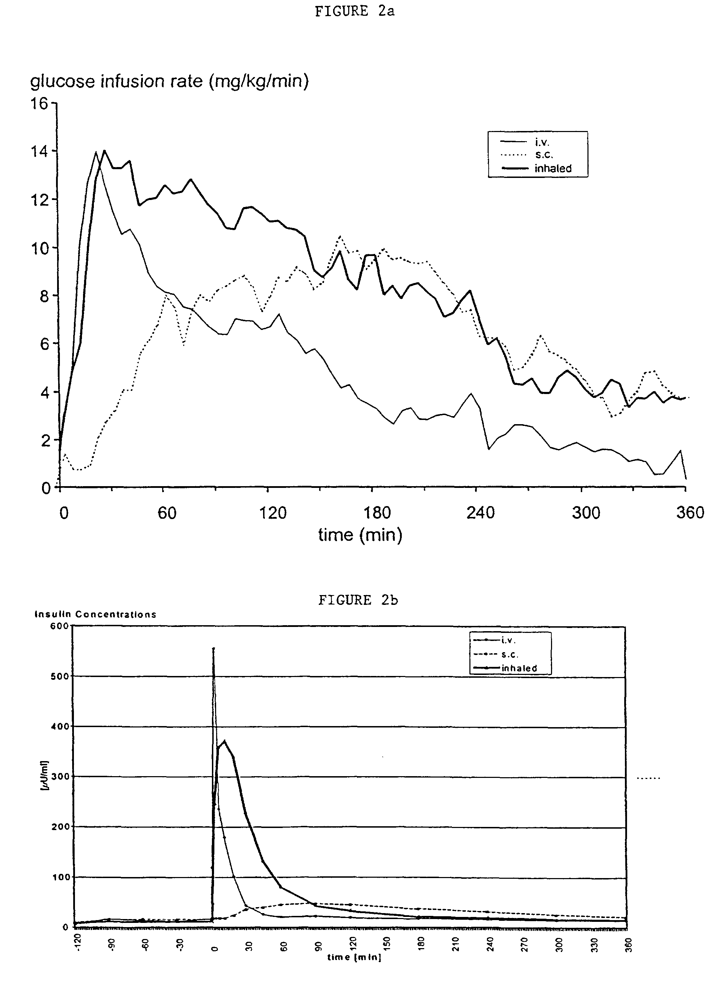

Bactericidal/permeability-increasing protein (BPI) is a protein isolated from the granules of mammalian polymorphonuclear neutrophils (PMNs), which are blood cells essential in defending a mammal against invading microorganisms. Human BPI has been isolated from PMNs by acid extraction combined with either ion exchange chromatography (Elsbach, 1979, *J*. *Biol. Chem*. 254: 11000) or *E. coli* affinity chromatography (Weiss *et al*., 1987, *Blood* 69: 652), and has bactericidal activity against gram-negative bacteria. The molecular weight of human BPI is approximately 55,000 daltons (55kD). The amino acid sequence of the entire human BPI protein and the nucleic acid sequence of DNA encoding BPI, have been reported by Gray *et al*., 1989, *J*. *Biol*. *Chem*. 264: 9505 (*see* Figure 1 in *Gray et al*.). The Gray *et al*. DNA and amino acid sequences are set out in SEQ ID NOS: 27 and 28 hereto.

The bactericidal effect of BPI was originally reported to be highly specific to sensitive gram-negative species. The precise mechanism by which BPI kills gram-negative bacteria is not yet known, but it is known that BPI must first attach to the surface of susceptible gram-negative bacteria. This initial binding of BPI to the bacteria involves electrostatic interactions between BPI, which is a basic (*i*.*e*., positively charged) protein, and negatively charged sites on lipopolysaccharides (LPS). LPS is also known as "endotoxin" because of the potent inflammatory response that it stimulates. LPS induces the release of mediators by host inflammatory cells which may ultimately result in irreversible endotoxic shock. BPI binds to Lipid A, the most toxic and most biologically active component of LPS.

BPI is also capable of neutralizing the endotoxic properties of LPS to which it binds. Because of its gram-negative bactericidal properties and its ability to bind to and neutralize LPS, BPI can be utilized for the treatment of mammals suffering from diseases and conditions initiated by infection with gram-negative bacteria whether the bacteria infect from outside the host or the bacteria infect from within the host (*i*.*e*., gut-derived), including conditions of bacteremia, endotoxemia, and sepsis. These properties of BPI make BPI particularly useful and advantageous for such therapeutic administration.

A proteolytic fragment corresponding to the amino-terminal portion of human BPI possesses the LPS binding and neutralizing activities and antibacterial activity of BPI holoprotein. In contrast to the amino-terminal portion, the carboxyl-terminal region of isolated human BPI displays only slightly detectable antibacterial activity and some endotoxin neutralizing activity (Ooi *et al*., 1991, *J*. *Exp. Med*. 174: 649). One BPI amino-terminal fragment, referred to as "rBPI₂₃" *(see* Gazzano-Santoro *et al*., 1992, *Infect*. *Immun*. 60: 4754-4761) has been produced by recombinant means as a 23kD protein and comprises an expression product of DNA encoding the first 199 amino acid residues of the human BPI holoprotein taken from Gray *et al*., *supra*, except that valine at position 151 is specified by GTG rather than GTC and residue 185 is glutamic acid (specified by GAG) rather than lysine (specified by AAG). Recombinant holoprotein, also referred to as rBPI, has also been produced having the sequence set out in SEQ ID NOS: 27 and 28 taken from Gray *et al*., *supra*, with the exceptions noted for rBPI₂₃, as also shown in U.S. Patent No. 5,198,541. An N-terminal fragment analog designated rBPI₂₁ or rBPI₂₁Δcys or rBPI (1-193) ala¹³² has been described in co-owned U.S. Patent No. 5,420,019 and corresponding International Publication No. WO 94/18323 (PCT/US94/01235). This analog comprises the first 193 amino acids of BPI holoprotein as set out in SEQ ID NOS: 27 and 28 but wherein the cysteine at residue number 132 is substituted with alanine, and with the exceptions noted for rBPI₂₃. rBPI₂₃, as well as the cysteine substitution analog designated rBPI₂₁, have been introduced into human clinical trials. Proinflammatory responses to endotoxin were significantly ameliorated when rBPI₂₃ was administered in humans challenged with endotoxin. (*See, e.g.,* co-owned U.S. Patent Nos. 5,643,875 and 5,753,620 and corresponding International Publication No. WO 95/19784 (PCT/US95/01151). In addition, rBPI₂₁ was administered in humans with meningococcemia and hemorrhage due to trauma. (*See*, *e*.*g*., U.S. Patent No. 5,888,977 and corresponding International Publication No. WO 97/42966 (PCT/US97/08016) and U.S. Patent No. 5,756,464 and corresponding International Publication No. WO 97/44056 (PCT/US97/08941).

Other endotoxin binding and neutralizing proteins and peptides are known in the art. One example is *Limulus* antilipopolysaccharide factor (LALF) from horseshoe crab amebocytes (Warren *et al*., 1992, *Infect. Immunol*. 60: 2506-2513). Another example is a cyclic, cationic lipopeptide from *Bacillus polymyxa*, termed Polymyxin B₁. Polymyxin B₁ is composed of six α,γ-diaminobutyric acid residues, one D-phenylalanine, one leucine, one threonine and a 6-methyloctanoyl moiety (Morrison and Jacobs, 1976, *Immunochem*. 13: 813-818) and is also bactericidal. Polymyxin analogues lacking the fatty acid moiety are also known, which analogues retain LPS binding capacity but are without appreciable bactericidal activity (Danner *et al*., 1989, *Antimicrob. Agents Chemother*. 33: 1428-1434). Similar properties have also been found with synthetic cyclized polymyxin analogues (Rustici *et al*., 1993, *Science* 259: 361-365).

Known antibacterial peptides include cecropins and magainins. The cecropins are a family of antibacterial peptides found in the hemolymph of lepidopteran insects (Wade *et al*., 1990, *Proc. Natl. Acad*. *Sci. USA* 87: 4761-4765), and the magainins are a family of antibacterial peptides found in *Xenopus* skin and gastric mucosa (Zasloff *et al*., 1988, *Proc. Natl. Acad. Sci*. *USA* 85: 910-913). These peptides are linear and range from about 20 to about 40 amino acids in length. A less active mammalian cecropin has been reported from porcine intestinal mucosa, cecropin P1 (Boman *et al*., 1993, *Infect. Immun.* 61: 2978-2984). The cecropins are generally reported to be more potent than the magainins in bactericidal activity and appear to have less mammalian cell cytotoxicity. The cecropins and magainins are characterized by a continuous, amphipathic α-helical region which is necessary for bactericidal activity. The most potent of the cecropins identified to date is cecropin A. The sequence of the first ten amino acids of the cecropin A has some homology with the BPI amino acid sequence 90-99 but does not share the motif of charged and uncharged amino acids specified by the BPI amino acid sequence 90-99. In addition, the other 27 amino acids of cecropin A are necessary for maximal bactericidal activity and there is no homology with BPI for those 27 amino acids. The magainins have minimal homology with the BPI amino acid sequence 90-99.

Of interest to the present application are the disclosures in PCT International Application PCT/US91/05758 [WO 92/03535] relating to compositions comprising BPI and an anionic compound, which compositions are said to exhibit (1) no bactericidal activity and (2) endotoxin neutralizing activity. Anionic compounds are preferably a protein such as serum albumin but can also be a polysaccharide such as heparin. In addition, Weiss *et al*., 1975, *J*. *Clin*. *Invest.* 55: 33-42, disclose that heparin sulfate and LPS block expression of the permeability-increasing activity of BPI. However, neither reference discloses that BPI actually binds to and/or neutralizes the biologic activities of heparin. Heparin binding does not necessarily imply heparin neutralization. For example, a family of heparin binding growth factors (HBGF) requires heparin as a cofactor to elicit a biological response. Examples of HBGF's include: fibroblast growth factors (FGF-1, FGF-2) and endothelial cell growth factors (ECGF-1, ECGF-2). Antithrombin III inhibition of clotting cascade proteases is another example of a heparin binding protein that requires heparin for activity and clearly does not neutralize heparin. Heparin binding proteins that do neutralize heparin (*e.g.*, platelet factor IV, protamine, and thrombospondin) are generally inhibitory of the activities induced by heparin binding proteins that use heparin as a cofactor.

Of particular interest to the present application are the heparin-related activities of BPI protein products. Specifically, BPI protein products have been shown to have heparin binding and heparin neutralization activities in co-assigned U.S. Patent Nos. 5,348,942; 5,639,727; 5,807,818; 5,837,678; 5,854,214 and corresponding International Publication No. WO 94/20128 (PCT/US94/02401). For example, rBPI₂₃ was shown to have high affinity for heparin (*see also*, Little *et al*., 1994, *J*. *Biol*. *Chem*. 269: 1865-1872, and has been administered in humans to neutralize heparin (*see, e.g.* U.S. Patent No. 5,348,942). These heparin binding and neutralization activities of BPI protein products are significant due to the importance of current clinical uses of heparin. Heparin is commonly administered in doses of up to 400 U/kg during surgical procedures such as cardiopulmonary bypass, cardiac catheterization and hemodialysis procedures in order to prevent blood coagulation during such procedures. When heparin is administered for anticoagulant effects during surgery, it is an important aspect of post-surgical therapy that the effects of heparin are promptly neutralized so that normal coagulation function can be restored. Currently, protamine is used to neutralize heparin. Protamines are a class of simple, arginine-rich, strongly basic, low molecular weight proteins. Administered alone, protamines (usually in the form of protamine sulfate) have anti-coagulant effects. When administered in the presence of heparin, a stable complex is formed and the anticoagulant activity of both drugs is lost. However, significant hypotensive and anaphylactoid effects of protamine have limited its clinical utility. Thus, due to its heparin binding and neutralization activities, BPI protein products have potential utility as a substitute for protamine in heparin neutralization in a clinical context without the deleterious side-effects which have limited the usefulness of the protamines. The additional antibacterial and anti-endotoxin effects of such BPI protein products would also be useful and advantageous in post-surgical heparin neutralization compared with protamine.

Additionally of particular interest, is the activity of BPI protein products to inhibit angiogenesis due in part to their heparin binding and neutralization activities. (*See, e.g.,* co-owned U.S. Patent Nos. 5,807,818 and 5,837,678 and corresponding International Publication No. WO 94/20128 (PCT/US94/02401). Angiogenesis, the growth of new blood vessels (neovascularization) is a complex phenomenon that involves growth factors, most of which have heparin as a co-factor. In adults, angiogenic growth factors are released as a result of vascular trauma (wound healing), immune stimuli (autoimmune disease), inflammatory mediators (prostaglandins) or from tumor cells. These factors induce proliferation of endothelial cells (which is necessary for angiogenesis) *via* a heparin-dependent receptor binding mechanism (*see* Yayon *et al*., 1991, *Cell* 64: 841-848). Angiogenesis is also associated with a number of other pathological conditions, including the growth, proliferation, and metastasis of various tumors; diabetic retinopathy, macular degeneration, retrolental fibroplasia, neovascular glaucoma, psoriasis, angiofibromas, immune and non-immune inflammation including rheumatoid arthritis, capillary proliferation within atherosclerotic plaques, hemangiomas, endometriosis and Kaposi's sarcoma. Thus, it would be desirable to inhibit angiogenesis in these and other instances, and the heparin binding and neutralization activities of BPI protein products, including peptides derived from or based on BPI, are useful to that end.

Heparin binding proteins fall into at least two classes. The first class consists of those proteins that utilize heparin as a co-factor in eliciting a specific response. These proteins include heparin-dependent growth factors (*e.g*., basic fibroblast growth factor, acidic fibroblast growth factor and vascular endothelial cell growth factor) which play a major role in angiogenesis. The second class includes proteins that neutralize the heparin-dependent response. BPI protein products, including peptides derived from BPI, have been identified as heparin neutralizing and anti-angiogenic agents. Several other heparin neutralizing proteins are also known to inhibit angiogenesis. For example, protamine is known to inhibit tumor-associated angiogenesis and subsequent tumor growth [*see* Folkman *et al*., 1992, *Inflammation: Basic Principles and Clinical Correlates,* 2d ed., (Galin *et al*., eds., Review Press, N.Y.), Ch. 40, pp. 821-839]. A second heparin neutralizing protein, platelet factor IV, also inhibits angiogenesis (*i*.*e*., is angiostatic). Another known angiogenesis inhibitor, thrombospondin, binds to heparin with a repeating serine/tryptophan motif instead of a basic amino acid motif (*see* Guo *et al*., 1992, *J*. *Biol*. *Chem*. 267: 19349-19355). Murine endostatin is also reported to bind heparin and inhibit angiogenesis (*see, e.g.,* Hohenester *et al*., 1998, *Embo J*. 17: 1656-1664; O'Reilly *et al*., 1997, *Cell* 88: 277-285).

Another utility of BPI protein products involves pathological conditions associated with chronic inflammation, which is usually accompanied by angiogenesis *(see, e.g.,* co-owned U.S. Patent No. 5,639,727). One example of a human disease related to chronic inflammation is arthritis, which involves inflammation of peripheral joints. In rheumatoid arthritis, the inflammation is autoimmune, while in reactive arthritis, inflammation is hypothesized to be associated with initial infection of the synovial tissue with pyogenic bacteria or other infectious agents followed by aseptic chronic inflammation in susceptible individuals. Folkman *et al*., 1992, *supra*; have also noted that many types of arthritis progress from a stage dominated by an inflammatory infiltrate in the joint to a later stage in which a neovascular pannus invades the joint and begins to destroy cartilage. While it is unclear whether angiogenesis in arthritis is a causative component of the disease or an epiphenomenon, there is evidence that angiogenesis is necessary for the maintenance of synovitis in rheumatoid arthritis. One known angiogenesis inhibitor, AGM1470, has been shown to prevent the onset of arthritis and to inhibit established arthritis in collagen-induced arthritis models (Peacock *et al*., 1992, *J*. *Exp. Med*. 175: 1135-1138). While nonsteroidal anti-inflammatory drugs, corticosteroids and other therapies have provided treatment improvements for relief of arthritis, there remains a need in the art for more effective therapies for arthritis and other inflammatory diseases.

Many additional utilities of BPI protein products, including rBPI₂₃ and rBPI₂₁, have been described due to the wide variety of biological activities of these products. For example, BPI protein products are bactericidal for gram-negative bacteria, as described in U.S. Patent Nos. 5,198,541 and 5,523,288. International Publication No. WO 94/20130 proposes methods for treating subjects suffering from an infection (*e.g.* gastrointestinal) with a species from the gram-negative bacterial genus *Helicobacter* with BPI protein products. BPI protein products also enhance the effectiveness of antibiotic therapy in gram-negative bacterial infections, as described in U.S. Patent No. 5,523,288 and International Publication No. WO 95/08344 (PCT/US94/11255). BPI protein products are also bactericidal for gram-positive bacteria and mycoplasma, and enhance the effectiveness of antibiotics in gram-positive bacterial infections, as described in U.S. Patent Nos. 5,578,572; 5,783,561 and 6,054,431 and International Publication No. WO 95/19180 (PCT/US95/00656). BPI protein products exhibit anti-fungal activity, and enhance the activity of other anti-fungal agents, as described in U.S. Patent No. 5,627,153 and International Publication No. WO 95/19179 (PCT/US95/00498), and further as described for anti-fungal peptides in U.S. Patent No. 5,858,974, which is in turn a continuation-in-part of U.S. Application No. 08/504,841 and corresponding International Publication Nos. WO 96/08509 (PCT/US95/09262) and WO 97/04008 (PCT/US96/03845). BPI protein products exhibit anti-protozoan activity, as described in U.S. Patent Nos. 5,646,114 and 6,013,629 and International Publication No. WO 96/01647 (PCT/US95/08624). BPI protein products exhibit anti-chlamydial activity, as described in co-owned U.S. Patent No. 5,888,973 and WO 98/06415 (PCT/US97/13810). Finally, BPI protein products exhibit anti-mycobacterial activity, as described in co-owned, co-pending U.S. Application No. 08/626,646, which is in turn a continuation of U.S. Application No. 08/285,803, which is in turn a continuation-in-part of U.S. Application No. 08/031,145 and corresponding International Publication No. WO 94/20129 (PCT/US94/02463).

The effects of BPI protein products in humans with endotoxin in circulation, including effects on TNF, IL-6 and endotoxin are described in U.S. Patent Nos. 5,643,875; 5,573,620 and 5,952,302 and corresponding International Publication No. WO 95/19784 (PCT/U595/01151).

BPI protein products are also useful for treatment of specific disease conditions, such as meningococcemia in humans (as described in co-owned U.S. Application No. 08/644,287 and U.S. Patent Nos. 5,888,977 and 5,990,086 and International Publication No. WO 97/42966 (PCT/US97/08016), hemorrhagic trauma in humans, (as described in U.S. Patent Nos. 5,756,464 and 5,945,399 and U.S. Application No. 09/293,107 and corresponding International Publication No. WO 97/44056 (PCT/US97/08941), burn injury (as described in U.S. Patent No. 5,494,896) ischemia/reperfusion injury (as described in U.S. Patent Nos. 5,578,568 and 6,017,881 and U.S. Application No. 09/416,828), and liver resection (as described in co-owned, co-pending U.S. Application No. 09/466,412 which is a continuation of U.S. Application No. 08/582,230, which is in turn a continuation of U.S. Application No. 08/318,357, which is in turn a continuation-in-part of U.S. Application No. 08/132,510, and corresponding International Publication No. WO 95/10297 (PCT/US94/11404).

BPI protein products are also useful in antithrombotic methods, as described in U.S. Patent Nos. 5,741,779 and 5,935,930 and U.S. Application No. 09/299,319 and corresponding International Publication No. WO 97/42967 (PCT/US7/08017).

WO-A-9 906 640 (Universiteit Van Amsterdam) discloses peptides with amino acid compositions such that the peptides are amphipatic, cationic, and form a stable α-helix and having the structures disclosed therein.

WO-A-9 505 393 (Morphosis) discloses LPS-binding peptides comprising an LPS-binding domain useful for the prevention or treatment of, for example, Gram-positive and Gram-negative bacterial sepsis.

There continues to exist a need in the art for new products that have one or more of the biological activities of BPI protein products, particularly products for use as heparin binding and neutralizing agents and for the inhibition of endothelial cell proliferation as well as inhibition of angiogenesis (normal or pathological). Advantageous therapeutic products that are peptide-based would ideally comprise small active sequences that are serum stable.

### SUMMARY OF THE INVENTION

This invention relates to compounds and compositions of small peptide-based constructs that are optionally derivatized with a hydrophobic moiety and that are 8-15 amino acid moieties in length, having a sequence that is derived from or based on reverse subsequences identified and selected from functional domain II (amino acids 65-99) of BPI and having at least one of the heparin-related biological activities of BPI, such as heparin binding, heparin neutralization, inhibition of endothelial cell proliferation and/or inhibition of angiogenesis. A reverse (or retro) sequence is inverted from the original (*e*.*g*., if an original sequence is A-B-C, the inverted sequence is C-B-A). Sequences herein are written in the conventional way, *i*.*e*., from the N-terminus to the C-terminus (left to right). Such peptide-based constructs, including derivatized constructs, according to a preferred aspect have reverse subsequences that consist of a minimum core sequence based on an amino acid motif derived from amino acids 99-92 of BPI. In a preferred embodiment the reverse subsequence is a substituted subsequence (for example, amino acids 99-92, 99-91, 99-90, 99-89, 99-88, 99-87, 99-86, or 99-85 wherein the substitutions are at 95 and 91). Additionally preferred is an 8-15 amino acid moiety sequence that has one or more D-amino acid moieties; in a most preferred sequence each or all of the amino acid moieties are D isomers.

Constructs (or compositions) disclosed herein consist of those that are 8-15 moieties in length having at least one of the heparin-related biological activities of BPI, such as heparin binding, heparin neutralizing, endothelial cell proliferation inhibiting, or antiangiogenic properties and comprise: (i) a sequence having the formula KLFR(naph-A)QAR₃ or (ii) a derivatized sequence of the formula R₁KLFR(naph-A)QAR₃, wherein
R₁ is any one of R₂―CH₂―, R₂―CH₂―CO―, R₂―CO―, R₂―SO_{y}―, or R₂―PO_{z}―;
wherein,
y=0-3;
z=1-4;
R₂ is a hydrophobic moiety that is a cyclic molecule having at least 3 carbon atoms, a heterocyclic molecule having at least 3 atoms, a functionalized cyclic molecule having at least 3 carbon atoms, or a functionalized heterocyclic molecule having at least three atoms.

As used herein, R₂ is a hydrophobic moiety that is any one of (a) an optionally substituted carbocyclic ring, saturated or partially or fully unsaturated, containing 3 to 8, preferably 5 or 6, carbon atoms; (b) an optionally substituted heterocyclic ring, saturated or partially or fully unsaturated, containing 3 to 8, preferably 5 or 6, atoms, wherein at least one atom is a heteroatom that is any one of oxygen, nitrogen, or sulfur; or (c) an optionally substituted bicyclic ring 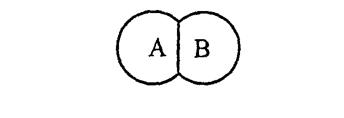 wherein the fused rings A and B, independently, are a 5- or 6-membered ring, saturated or partially or fully unsaturated, and comprise carbon atoms and optionally one to three heteroatoms selected from oxygen, sulfur, or nitrogen; where there is more than one heteroatom, each may be the same or different.

As used herein, substituent R₂ can be a monocyclic or bicyclic ring, either a carbocycle or a heterocycle. Bicyclic R₂ groups can contain two aliphatic rings, two aromatic rings, or one aliphatic and one aromatic ring. Heterocycles contain at least one, and up to three, atoms selected from the group consisting of oxygen, nitrogen, or sulfur; where there is more than one heteroatom, each heteroatom may be the same or different. An R₂ substituent can be aliphatic, saturated or partially or fully unsaturated (*i*.*e*., cycloalkyl, cycloalkenyl, heterocycloalkyl, or heterocycloalkylenyl), or can be aromatic (*i*.*e*., aryl or heteroaryl). The R₂ substituent is optionally substituted with one to three moieties, for example, C₁₋₆alkyl, halo, C₁₋₆alkoxy, acetyl, hydroxy, or the like; where there is more than one heteroatom, each heteroatom may be the same or different.

As used herein and as commonly used in the art, the term "aryl" is defined as a monocyclic or bicyclic aromatic group, *e.g*., phenyl or naphthyl, which can be unsubstituted or substituted. Likewise, the term "heteroaryl" is defined herein as a monocyclic or bicyclic ring system containing one or two aromatic rings and at least one nitrogen, oxygen, or sulfur atom. Where there is more than one heteroatom, each heteroatom may be the same or different. The heteroaryl can be unsubstituted or substituted, for example, with one or more, in particular one to three, substituents. Where there is more than one heteroatom, each heteroatom may be the same or different. Examples of heteroaryl groups include thienyl, furyl, pyridyl, oxazolyl, quinolyl, isoquinolyl, indolyl, triazolyl, isothiazolyl, isoxazolyl, imidizolyl, benzothiazolyl, pyrazinyl, pyrimidinyl, thiazolyl, or thiadiazolyl.

As used herein and as commonly used in the art, the term "cycloalkyl" is defined as a cyclic C₃-C₈ hydrocarbon group, e.g., cyclopropyl, cyclobutyl, cyclohexyl, or cyclopentyl. The term "heterocycloalkyl" is similarly defined, except the ring contains at least one, preferably one to three, heteroatoms. Where there is more than one heteroatom, each heteroatom may be the same or different. Nonlimiting examples of heterocycloalkyl rings include 1,3-dioxolane, 2-pyrazoline, pyrazolidine, pyrrolidine, a pyrroline, 2H-pyran, 4H-pyran, morpholine, thiomorpholine, piperidine, 1,4-dithiane, and 1,4-dioxane. The terms "cycloalkenyl" and "heterocycloalkenyl" are similarly defined, except the ring is unsaturated.

In exemplary embodiments, R₂ may be a hydrophobic moiety that is any one of biotin, 2-biphenylene, 2-anthraquinone, 2-benzofuran, 2-indole, 1-isoquinoline, hydroxyphenyl, 2-quinoline, 1-[3-(3,4-dihydroxycinnamoyl)-1,3,4,5-tetrahydroxycyclohexyl], 1-(3,5-dichloro-2-hydroxyphenyl), 1-(3,5-diiodo-2-hydroxyphenyl), 1-(3,5-dinitro-2-hydroxyphenyl), 1-(4-azido-2-hydroxyphenyl), 4-biphenyl, 2-biphenyl, 1-naphthyl, 2-naphthyl, 3-amino-2-naphthyl, 3-chloro-2-nitrophenyl, 3,4-dihydroxyphenyl, 3,4,5-trihydroxyphenyl, 2-chloro-3-nitrophenyl, 5-azido-2-nitrophenyl, 3-amino-2-pyrazyl, 2-benzyloxycarbonyl-ethyl, 2-thienyl, 2-(3,4-dihydroxyphenyl)ethylene, 5-bromo-3-indolemethylene, 2-(4-hydroxy-3-methoxyphenyl)ethylene, 2-(3-chlorophenyl)ethylene, 2-pyrazyl, 4-imidazolyl, 2-imino-1-imidazolidyl, pyridyl, 3-piperidyl, 4-piperidyl, fluorescein, 2-(4-amino-3,5,6-trichloro-pyridyl), 3-(2-chloro-6-fluorophenyl)-5-methylisoxazolyl, or 4-azido-phenyl.

R₃ is any one of K, K(naph-A), K(naph-A)K, K(naph-A)KG, K(naph-A)KGS, K(naph-A)KGSI, K(naph-A)KGSIK or K(naph-A)KGSIKI;
wherein the carboxyl terminal group is amidated or nonamidated,
and, optionally, comprising at least one conservative substitution of amino acid moieties; wherein the composition is not k-l-f-r (naph-a)-q-a-k-(naph-a)-k-g-s-i-k-i. Preferably, the composition comprises two or more conservative substitutions of amino acid moieties.

The constructs or compositions of the invention, including derivatized constructs or compositions, preferably comprise constructs or compositions wherein the first two amino-terminal amino acid moieties are D-amino acid moieties and the last two carboxy-terminal amino acid moieties are D-amino acid moieties.

Also disclosed are methods for the preparation of medicaments for neutralizing heparin in a mammal that has been administered an exogenous heparin compound (including heparin or heparinoid substances, such as low molecular weight heparins) comprising the step of administering to said mammal an amount of the composition of the invention effective to neutralize the anticoagulant effect of the exogenous heparin compound, preferably in an amount effective to return the clotting time of said mammal to normal; methods of preparation of medicaments for inhibiting endothelial cell proliferation in a mammal in need thereof by administering to said mammal an amount of the compositions of the invention effective to inhibit endothelial cell proliferation; methods of preparation of a medicament for inhibiting angiogenesis in a mammal in need thereof by administering to said mammal an amount of such compositions effective to inhibit angiogenesis, including angiogenesis in the eye; methods for the preparation of a medicament for treating a mammal suffering from a disorder involving angiogenesis, including a chronic inflammatory disease, such as rheumatoid or reactive arthritis, and including the growth, proliferation or metastasis of tumor cells.

Additional properties or activities of such constructs or compositions, including those derivatized according to the invention may include LPS binding, LPS neutralization, and/or antimicrobial activity and/or any other previously known activity or property of BPI protein products. Although three functional domains of BPI were previously reported and include: domain I, encompassing the amino acid sequence of BPI from about amino acid 17 to about amino acid 45; domain II, encompassing the amino acid sequence of BPI from about amino acid 65 to about amino acid 99; and domain III, encompassing the amino acid sequence of BPI from about amino acid 142 to about amino acid 169, biologically active reverse (or retro) sequences have not been previously reported based on subsequences of domain II. Thus, such peptide-based reverse (retro) sequence constructs according to the invention, which preferably comprise selected D-amino acid moieties, are particularly useful as therapeutic agents.

Another aspect of the invention provides methods for identifying a derivatized peptide sequence derived from or based on the sequence identified and selected from Domain II of bactericidal/permeability-increasing protein (BPI) having biological activity and epithelial absorption of at least 0.001% comprising the steps of:
(a) derivatizing a peptide sequence based on a sequence, subsequence, reverse sequence or reverse subsequence of Domain II of BPI through covalent linkage of a hydrophobic moiety or moieties at the N-terminus, C-terminus or within said peptide sequence;
(b) measuring the activity of said derivatized peptide sequence obtained in step (a) wherein the activity is any one or more of heparin binding, heparin neutralizing, endothelial cell proliferation inhibiting, antiangiogenic LPS binding, LPS neutralizing or antimicrobial properties; and
(c) measuring the epithelial absorption of said derivatized peptide sequence obtained in step (a).

Peptide sequences particularly suitable for derivatization in step (a) are peptides of minimal length necessary to retain biological activity (e.g. 8 to 15 amino acid moieties).

Such methods include a method for designing and identifying a biologically active derivatized peptide-based sequence, prophylactic or therapeutic medicament derived from or based on the peptide sequence identified and selected from BPI or a fragment thereof with epithelial absorption of at least 0.001%, said method comprising the steps of:
(a) identifying a target peptide sequence derived from or based on the polypeptide sequence of BPI or a fragment thereof which exhibits activity *in vitro* or *in vivo* wherein the activity is any one or more of heparin binding, heparin neutralizing, endothelial cell proliferation inhibiting, antiangiogenic LPS binding, LPS neutralizing or antimicrobial properties;
(b) constructing a library of minimum length, activity retaining peptide sequences (MinLARPS) by substituting or deleting amino acid moieties within said target peptide sequence;
(c) measuring the activity of said MinLARPS to determine the minimum number of residues necessary to retain activity of at least 1% of that of said target polypeptide sequence wherein the activity is any one or more of heparin binding, heparin neutralizing, endothelial cell proliferation inhibiting, antiangiogenic LPS binding, LPS neutralizing or antimicrobial properties;
(d) measuring epithelial absorption of said MinLARPS in *in vivo* or *in vitro* assays to identify which of said MinLARPS retain epithelial absorption of at least 0.001%;
(e) synthesizing derivatized MinLARPS by chemically modifying said MinLARPS through covalent linkage of a hydrophobic moiety or moieties linked at the N-terminus, C-terminus, or within the sequence of said MinLARPS;
(f) repeating steps (c) and (d) with said derivatized MinLARPS.

Further aspects of the invention include a construct or composition of the invention (including those derivatized according to the invention) for use in therapy, as well as the use of such a construct or composition for the manufacture of a medicament for binding and/or neutralizing an exogenous or therapeutically administered heparin compound, or for binding and/or neutralizing heparin, or for treating a heparin-related or heparin-mediated disorder, condition or disease, or for the manufacture of a medicament for antimicrobial activity for binding and/or neutralizing LPS, or for treating an infection or a disorder associated with endotoxin.

Also contemplated by the present invention is a pharmaceutical composition comprising a construct or composition of the present invention, and a pharmaceutically acceptable adjuvant, diluent or carrier.

Also provided is an in vitro method of neutralizing the anticoagulant effect of heparin comprising contacting the heparin with a construct or composition of the invention.

### DETAILED DESCRIPTION

The present invention relates to biologically active novel compounds (or compositions) that are 8 to 15 amino acid moieties in length, having a sequence that is derived from or based on a reverse subsequence identified and selected from functional domain II of BPI. Constructs include non-derivatized sequences as well as sequences that are derivatized by covalent linkage of a hydrophobic moiety. Preferred are constructs with sequences that contain D-amino acid moieties. Particularly preferred are constructs with sequences where the D-amino acid moieties are positioned as the first two amino-terminal and last two carboxy-terminal moieties of the sequence. Such constructs are particularly useful for the treatment of heparin-related or heparin-mediated disorders, diseases or conditions. "Treatment" as used herein encompasses both prophylactic and therapeutic treatment. Treatment of mammals, including humans, is contemplated.

As used herein, "amino acid moiety" includes typical and atypical amino acid compounds (including derivatized amino acids and amino acid analogs). "Conservative" substitutions of one amino acid for another are substitutions of amino acids having similar structural and/or chemical properties, and are generally based on similarities in polarity, charge, hydrophobicity, hydrophilicity and/or the amphipathic nature of the residues involved. Polar acidic amino acids include aspartic acid and glutamic acid. As a general rule, as the similarity between the amino acids being substituted decreases, the likelihood that the substitution will affect activity increases.

For the purposes of this invention, the term "functional domain" is intended to designate a region of the amino acid sequence of BPI that exhibits one or more of the biological activities of BPI. These functional domains of BPI were defined by the activities of proteolytic cleavage fragments, overlapping 15-mer peptides and other synthetic peptides. Domain I has been defined as the amino acid sequence of BPI comprising from about amino acid 17 to about amino acid 45. Initial peptides based on this domain were moderately active in both the inhibition of LPS-induced LAL activity and in heparin binding assays, and did not exhibit significant antibacterial activity. Domain II has been defined as the amino acid sequence of BPI comprising from about amino acid 65 to about amino acid 99. Initial peptides based on this domain exhibited high LPS and heparin binding capacity and exhibited significant antibacterial activity. Domain III has been defined as the amino acid sequence of BPI comprising from about amino acid 142 to about amino acid 169. Initial peptides based on this domain exhibited high LPS and heparin binding activity, and exhibited surprising antimicrobial activity, including antifungal and antibacterial (including, e.g., anti-gram-positive and anti-gram-negative) activity.

For the purposes of this invention, the term "biological activity of BPI" is intended to include, but is not limited to one or more of the biological activities or properties of a human bactericidal/permeability-increasing (BPI) protein product, including, for example, a recombinant BPI holoprotein such as rBPI (SEQ ID NO: 28), an amino-terminal fragment of BPI such as rBPI₂₃, and analogs that are mutated amino-terminal fragments of BPI such as rBPI₂₁Δcys or rBPI (10-193) C132A (also designated rBPI (10-193) ala¹³²) and including any one or more of the known activities of the BPI protein products discussed above. Specifically included is a biological activity of any peptide-based construct of this invention that is between 0.1 and 10 times the activity of BPI or of a corresponding peptide encompassing a corresponding functional domain of BPI. The term "biological activity of BPI" is intended to include, but is not limited to an activity of heparin binding, heparin neutralization, inhibition of endothelial cell proliferation or inhibition of angiogenesis (*e.g.,* inhibition of *in vivo* neovascularization such as that associated with metastatic tumors and chronic inflammatory disease states). Also included in this definition of "biological activity of BPI" is an activity of LPS binding, LPS neutralization, or antimicrobial activity. Also expressly included in this definition of the "biological activity of BPI" is a biological activity, for example antimicrobial activity, that is qualitatively different than the activity of BPI or the corresponding peptide encompassing the entire corresponding domain of BPI. For example, such qualitative differences include differences in the spectrum of bacteria or other microorganisms against which the peptide is effective, relative to the amino acid sequence of the corresponding functional domain of BPI. This definition thus encompasses antimicrobial activities, such as antibacterial activity (e.g., against gram-negative bacteria, gram-positive bacteria, mycobacteria and chlamydia) and antifungal activity (*e.g.,* against species of *Candida, Aspergillus, Cryptococcus, Histoplasma, Coccidioides, Blastomyces, Basidiobolus, Conidiobolus, Rhizopus, Rhizomucor, Mucor, Absidia, Mortierella, Cunninghamella, Saksenaea, Fusarium, Trichophyton, Trichosporon, Microsporum, Epidermophyton, Scytalidium, Malassezia, Actinomyceies, Sporothrix and Penicillium*), as well as anti-protozoal activity

For the purposes of this invention, the term "derivatized" in the context of a "derivatized construct" or "derivatized composition" refers to a peptide-based construct or composition comprising sequences covalently linked to a hydrophobic moiety. Preferably, the hydrophobic moiety is covalently linked to the N-terminus of the sequence. Preferably, the hydrophobic moiety is R₂ as defined herein.

Peptide-based constructs, including those suitable for derivatization, include sequences derived from or based on reverse substituted subsequences from functional domain II of human BPI (*e*.*g*., amino acids 99-92, 99-91, 99-90, 99-89, 99-88, 99-87, 99-86, or 99-85 wherein the substitutions are at 75 and 91)), preferably D-amino acid moiety sequences. Embodiments of such constructs according to the invention include the following exemplary constructs [single-letter abbreviations for amino acids can be found in G. Zubay, *Biochemistry* (2d. ed.), 1988 (MacMillen Publishing: N.Y.), p.33]:

Exemplary derivatized peptide-based constructs include:

As used herein, "BPI protein product" includes naturally and recombinantly produced BPI protein; natural, synthetic), and recombinant biologically active polypeptide fragments of BPI protein; biologically active polypeptide variants of BPI protein or fragments thereof, including hybrid fusion proteins and dimers; biologically active polypeptide analogs of BPI protein or fragments or variants thereof, including cysteine-substituted analogs; and BPI-derived peptides. BPI protein products may be generated and/or isolated by any means known in the art. U.S. Patent No. 5,198,541, discloses recombinant genes encoding, and methods for expression of, BPI proteins including recombinant BPI holoprotein, referred to as rBPI and recombinant fragments of BPI. U.S. Patent No. 5,439,807 and corresponding International Publication No. WO 93/23540 (PCT/US93/04752) disclose novel methods for the purification of recombinant BPI protein products expressed in and secreted from genetically transformed mammalian host cells in culture and discloses how one may produce large quantities of recombinant BPI products suitable for incorporation into stable, homogeneous pharmaceutical preparations.

Biologically active fragments of BPI (BPI fragments) include biologically active molecules that have the same or similar amino acid sequence as a natural human BPI holoprotein, except that the fragment molecule lacks amino-terminal amino acids, internal amino acids, and/or carboxy-terminal amino acids of the holoprotein without loss of one or more of the biological activities or immunological properties of BPI. Nonlimiting examples of such fragments include an N-terminal fragment of natural human BPI of approximately 25 kD, described in Ooi et al., 1991, *J*. *Exp*. *Med., 174*:649, and the recombinant expression product of DNA encoding N-terminal amino acids from 1 to about 193 to 199 of natural human BPI, described in Gazzano-Santoro et al., 1992, *Infect. Immun*. *60*:4754-4761, and referred to as rBPI₂₃. In that publication, an expression vector was used as a source of DNA encoding a recombinant expression product (rBPI₂₃) having the 31-residue signal sequence and the first 199 amino acids of the N-terminus of the mature human BPI, as set out in Figure 1 of Gray et al., *supra*, except that valine at position 151 is specified by GTG rather than GTC and residue 185 is glutamic acid (specified by GAG) rather than lysine (specified by AAG). Recombinant holoprotein (rBPI) has also been produced having the sequence (SEQ ID NOS: 27 and 28) set out in Figure 1 of Gray et al., *supra,* with the exceptions noted for rBPI₂₃ and with the exception that residue 417 is alanine (specified by GCT) rather than valine (specified by GTT). An analog of an N-terminal fragment consisting of residues 10-193 of BPI has been described in U.S. Patent No. 6,013,631 and corresponding International Publication No. WO 99/66044 (PCT/US99/13860). Other examples include dimeric forms of BPI fragments, as described in U.S. Patent No. 5,447,913 and corresponding International Publication No. WO 95/24209 (PCT/US95/03125).

Biologically active variants of BPI (BPI variants) include but are not limited to recombinant hybrid fusion proteins, comprising BPI holoprotein or biologically active fragment thereof and at least a portion of at least one other polypeptide, and dimeric forms of BPI variants. Examples of such hybrid fusion proteins and dimeric forms are described in U.S. Patent No. 5,643,570 and corresponding International Publication No. WO 93/23434 (PCT/US93/04754) and include hybrid fusion proteins comprising, at the amino-terminal end, a BPI protein or a biologically active fragment thereof and, at the carboxy-terminal end, at least one constant domain of an immunoglobulin heavy chain or allelic variant thereof.

Biologically active analogs of BPI (BPI analogs) include but are not limited to BPI protein products wherein one or more amino acid residues have been replaced by a different amino acid without loss of one or more of the biological activities or immunological properties of BPI. For example, U.S. Patent No. 5,420,019 and corresponding International Publication No. WO 94/18323 (PCT/US94/01235) discloses polypeptide analogs of BPI and BPI fragments wherein a cysteine residue is replaced by a different amino acid. A stable BPI protein product described by this application is the expression product of DNA encoding from amino acid 1 to approximately 193 or 199 of the N-terminal amino acids of BPI holoprotein, but wherein the cysteine at residue number 132 is substituted with alanine and is designated rBPI₂₁Δcys or rBPI₂₁. Production of this N-terminal analog of BPI, rBPI₂₁, has been described in Horwitz et al., 1996, *Protein Expression Purification*, *8*:28-40. Similarly, a fragment consisting of residues 10-193 of BPI in which the cysteine at position 132 is replaced with an alanine (designated ''rBPI(10-193)C132A" or "rBPI(10-193)ala¹³²") has been described in U.S. Patent No. 6,013,631 and corresponding International Publication No. WO 99/66044 (PCT/US99/13860). Other examples include dimeric forms of BPI analogs; *e*.*g*. U.S. Patent No. 5,447,913 and corresponding International Publication No. WO 95/24209 (PCT/US9S/03125).

Other BPI protein products are peptides derived from or based on BPI produced by synthetic or recombinant means (BPI-derived peptides), such as those described in International Publication No. WO 97/04008 (PCT/US96/03845), which corresponds to U.S. Patent No. 5,858,974 and International Publication No. WO 96/08509 (PCT/US95/09262), which corresponds to U.S. Patent Application No. 09/365,539, and International Publication No. WO 95/19372 (PCT/US94/10427), which corresponds to U.S. Patent Nos. 5,652,332 and 5,856,438, and International Publication No. WO94/20532 (PCT/US94/02465), which corresponds to U.S. Patent No. 5,763,567 which is a continuation of U.S. Patent No. 5,733,872, which is a continuation-in-part of U.S. Application No. 08/183,222, which is a continuation-in-part of U.S. Application No. 08/093,202 (corresponding to International Publication No. WO 94/20128 (PCT/US94/02401)), which is a continuation-in-part of U.S. Patent No. 5,348,942, as well as International Publication No. WO 97/35009 (PCT/US97/05287), which corresponds to U.S. Patent No. 5,851,802.

The present invention defines novel peptide-based constructs, including derivative constructs, that may be encompassed in the definition of BPI protein products.

The administration of BPI protein products is preferably accomplished with a pharmaceutical composition comprising a BPI protein product and a pharmaceutically acceptable diluent, adjuvant, or carrier. The BPI protein product may be administered without or in conjunction with known surfactants or other therapeutic agents. A stable pharmaceutical composition containing BPI protein products (e.g., rBPI₂₃) comprises the BPI protein product at a concentration of I mg/ml in citrate buffered saline (5 or 20 mM citrate, 150 mM NaCl, pH 5.0) comprising 0.1% by weight of poloxamer 188 (PLURONIC® F-68, BASF Wyandotte, Parsippany, NJ) and 0.002% by weight of polysorbate 80 (TWEEN® 80, ICI Americas Inc., Wilmington, DE). Another stable pharmaceutical composition containing BPI protein products (e.g., rBPI₂₁) comprises the BPI protein product at a concentration of 2 mg/ml in 5 mM citrate, 150 mM NaCl, 0.2% poloxamer 188 and 0.002% polysorbate 80. Such preferred combinations are described in U.S. Patent Nos. 5,488,034; 5,696,090; 5,932,544; 5,955,427; 6,066,620; and 6,057,293 and corresponding International Publication No. WO 94/17819 (PCT/US94/01239). As described in U.S. Patent No. 5,912,228, which is in turn a continuation-in-part of U.S. Application No. 08/530,599, which is in turn a continuation-in-part of U.S. Application No. 08/372,104, and corresponding International Publication No. WO 96/21436 (PCT/US96/01095) other poloxamer formulations of BPI protein products with enhanced activity may be utilized. Peptide-based constructs may be formulated like other BPI protein products or may be formulated in saline or a physiological buffer.

Therapeutic compositions comprising BPI protein product (including the peptide-based constructs, including derivatized constructs, or compositions comprising such constructs of the invention) may be administered systemically or topically. Systemic routes of administration include oral, intravenous, intramuscular or subcutaneous injection (including into a depot for long-term release), intraocular and retrobulbar, intrathecal, intraperitoneal (e.g. by intraperitoneal lavage), intrapulmonary (using powdered drug, or an aerosolized or nebulized drug solution), or transdermal. Topical routes include administration in the form of salves, ophthalmic drops, ear drops, or irrigation fluids (for, e.g., irrigation of wounds).

When given parenterally, BPI protein product compositions are generally injected in doses ranging from 1 µg/kg to 100 mg/kg per day, preferably at doses ranging from 0.1 mg/kg to 20 mg/kg per day. The treatment may continue by continuous infusion or intermittent injection or infusion, at the same, reduced or increased dose per day for, *e.g.*, 1 to 3 days, and additionally as determined by the treating physician.

Those skilled in the art can readily optimize effective dosages and administration regimens for therapeutic compositions comprising BPI protein product (including the constructs or compositions of the present invention), as determined by good medical practice and the clinical condition of the individual subject.

The constructs, including derivatized constructs, or compositions comprising such constructs of the invention may be used in any of the therapeutic uses for which BPI products are known to be effective, including those described above and are expected to have epithelial cell absorption of at least about 0.001%, or more preferably at least about 0.01%, 0.1%, 1%, 10% or 20% or more. Constructs and compositions of the invention, particularly those that are derivatized, can optionally be tested for their epithelial absorption properties by any assay known in the art, including the oral absorption or transport screening assays described in Examples 6, 7 or 8. The constructs are particularly useful in methods for binding and neutralizing exogenous heparin, methods for inhibiting endothelial cell proliferation, treating disorders associated with endothelial cell proliferation, methods for inhibiting angiogenesis, and treating disorders associated with or involving angiogenesis, but may also be useful for other diseases or conditions treatable due to other biological activities of BPI, including infections or disorders associated with endotoxin and specifically including any of the diseases or conditions described herein or known in the art with reference to BPI protein products.

Exogenous heparin compounds are commonly administered during surgical procedures requiring anticoagulation, such as cardiopulmonary bypass, cardiac catheterization or angioplasty, and hemodialysis. Exogenous heparin compounds are also administered to patients at risk of or suffering from thrombosis, e.g. patients suffering from deep venous thrombosis, acute myocardial infarction, stroke, or pulmonary embolism.

Angiogenesis-associated disorders are disorders in which angiogenesis plays a role in the initiation or progression of disease. Angiogenesis is involved in a number of conditions, illustrated below, and inhibition of angiogenesis is expected to be effective for treating any of these conditions (including inhibiting progression of the disease and ameliorating signs and symptoms of the disease).

Use of the constructs or compositions of the invention, including those derivatized according to the invention, in preparation of a medicament for any of these therapeutic uses is also contemplated.

Angiogenesis is of considerable importance in cancer conditions because new vessel production is required to support the rapid growth of cancer cells. Inhibition of angiogenesis thus may promote tumor regression in adult and pediatric oncology, including reducing growth of solid tumors/malignancies, locally advanced tumors, metastatic cancer, human soft tissue sarcomas, cancer metastases, including lymphatic metastases, blood cell malignancies, effusion lymphomas (body cavity based lymphomas), lung cancer, including small cell carcinoma, non-small cell cancers, breast cancer, including small cell carcinoma and ductal carcinoma, gastrointestinal cancers, including stomach cancer, colon cancer, colorectal cancer, polyps associated with colorectal neoplasia, pancreatic cancer, liver cancer, urological cancers, including bladder cancer, prostate cancer, malignancies of the female genital tract, including ovarian carcinoma, uterine endometrial cancers, and solid tumors in the ovarian follicle, kidney cancer, including renal cell carcinoma, brain cancer, including intrinsic brain tumors, neuroblastoma, astrocytic brain tumors, gliomas, metastatic tumor cell invasion in the central nervous system, bone cancers, including osteomas, skin cancers, including malignant melanoma, tumor progression of human skin keratinocytes, and squamous cell cancer, hemangiopericytoma, and Kaposi's sarcoma.

Angiogenesis also plays a role in chronic inflammation, including chronic pancreatitis, dermatosis associated with chronic inflammation, including psoriasis, cirrhosis, asthma, multiple sclerosis, arthritis, including rheumatoid arthritis, reactive arthritis and chronic inflammatory arthritis, autoimmune disorders, including vasculitis, glomerulonephritis, experimental allergic encephalomyelitis (EAE), lupus, myasthenia gravis, ulcerative colitis, Crohn's disease, inflammatory bowel disease, chronic inflammation associated with hemodialysis, granulocyte transfusion associated syndrome; rejection reactions after allograft and xenograft transplantation, including graft versus host disease; and other chronic inflammatory disorders.

Angiogenesis in the eye is involved in ocular neovascularization, proliferative retinopathy, retrolental fibraplasia, macular degeneration, neovascular glaucoma and diabetic ocular disease, in particular, diabetic iris neovascularization and retinopathy.

Coronary atheroma are highly vascularized by a fragile capillary network, and rupture of these newly formed capillaries when they are exposed to high intravascular pressures may lead to hemorrhage into atherosclerotic plaques and coronary occlusion. Inhibition of angiogenesis thus may reduce the growth of atherosclerotic plaques and may be useful in the treatment of atherosclerosis, ischemic heart disease, myocardial infarction, coronary heart disease, restenosis, particularly following balloon angiography, neointimal hyperplasia, disruption of intercellular junctions in vascular endothelium, hypertension, vessel injury, arterial ischemia, arterial stenosis, peripheral vascular disease, stroke

Angiogenesis also occurs during the female reproductive cycle and is involved in endometriosis, uterine fibroids, other conditions associated with dysfunctional vascular proliferation (including endometrial microvascular growth) during the female reproductive cycle.

Angiogenesis is also involved in abnormal vascular growth, including cerebral arteriovenous malformations (AVMs), angiofibronas, and hemangionas.

Concurrent administration of other therapeutic agents appropriate for the condition being treated (e.g., other agents that inhibit angiogenesis or cancer therapeutic agents if indicated) is also contemplated.

"Concurrent administration," or "co-administration," as used herein includes administration of the agents, in conjunction or combination, together, or before or after each other. The BPI protein product and second agent(s) may be administered by different routes. For example, the BPI protein product may be administered intravenously while the second agent(s) is(are) administered intravenously, intramuscularly, subcutaneously, orally or intraperitoneally. The BPI protein product and second agent(s) may be given sequentially in the same intravenous line or may be given in different intravenous lines. Alternatively, the BPI protein product may be administered in a special form for gastric delivery, while the second agent(s) is(are) administered, e.g., orally. The formulated BPI protein product and second agent(s) may be administered simultaneously or sequentially, as long as they are given in a manner sufficient to allow all agents to achieve effective concentrations at the site of action.

Other aspects and advantages of the present invention will be understood upon consideration of the following illustrative examples wherein: Example 1 addresses preparation and purification of peptide-based constructs, including derivatized constructs; Example 2 addresses *in vitro* activity of peptide-based constructs, including derivatized constructs, in an endothelial cell proliferation assay; Example 3 addresses *in vivo* testing of the anti-angiogenic activities of peptide-based constructs, including derivatized constructs; Example 4 addresses the *in vivo* testing of peptide-based constructs, including derivatized constructs, in models of chronic inflammatory disease states; Example 5 addresses testing of peptide-based constructs, including derivatized constructs, in a malignant melanoma metastasis model; Example 6 addresses the *in vitro* testing of peptide-based constructs, including derivatized constructs, in oral absorption screening assays; Example 7 addresses the *in vivo* testing of peptide-based constructs, including derivatized constructs, for oral absorption; Example 8 addresses *in vivo* testing of peptide-based constructs, including derivatized constructs, for oral activity; Example 9 addresses the *in vitro* and *in vivo* testing of peptide-based constructs, including derivatized constructs, in retinal neovascularization models; Example 10 addresses the testing of peptide-based constructs, including derivatized constructs, for heparin neutralization; and Example 11 addresses the testing of peptide-based constructs, including derivatized constructs, in additional activity assays for BPI protein products.

### EXAMPLE 1

### PREPARATION AND PURIFICATION OF PEPTIDE-BASED CONSTRUCTS

This example addresses the preparation and purification of peptide-based constructs, including derivatized constructs.

Peptide-based constructs may be prepared according to a variety of synthetic procedures. For example, BPI-derived peptides have been prepared by solid phase peptide synthesis as described in co-assigned U.S. Patent Application No. 08/183,222 and U.S. Patent No. 5,733,872, according to the methods of Merrifield, 1963, *J*. *Am Chem*. *Soc. 85*: 2149 and Merrifield *et al*., 1966, *Anal. Chem*., *38*: 1905-1914 using an Applied Biosystems, Inc. Model 432 peptide synthesizer.

Alternatively, BPI-derived peptides have been synthesized on a larger scale using solid phase peptide synthesis on an Advanced Chemtech (ACT-Model 357 MPS) synthesizer utilizing a 1-Fluorenylmethyl-oxycarbonyl (Fmoc) protection strategy with a double coupling procedure employing N,N-diisopropylcarbodiimide (DIC or DIPCDI)/1-hydroxybenzotriazole (HOBt) and 2-(1-H-benzotriazol-1-yl)-1,1,3,3,-tetramethyluronium hexa-fluorophosphate (HBTU)/HOBt/ diisopropylethylamine (DIEA) as described in U.S. Patent No. 5,858,974.

The solid support used in the synthesis of peptide-based constructs of the present invention was a polystyrene resin with 1% divinylbenzene (DVB) crosslinking and an 4-(2',4'-dimethoxyphenyl-Fmoc-aminomethyl)-phenoxy (Fmoc-Rink amide) linker with a substitution rate of 0.56 mmoles/gram. A scale between 0.1 grams and 5 grams of starting resin may be used, and was generally 0.25 grams for the synthetic peptide-based constructs described herein.

For such syntheses, dimethylformamide (DMF) was the primary solvent with a 50/50 solution of piperidine/DMF used for Fmoc deprotection in three consecutive treatments of 1, 5, and 10 minutes, respectively. A double coupling procedure was used in each cycle with a 4:1 amino acid to peptide ratio used in each coupling. The amino acids were dissolved in a 0.5M HOBt solution in N-methylpyrrolidinone (NMP) at a concentration also of 0.5M. For the first coupling, an equimolar (to amino acid) amount of a 0.5M solution of diisopropylcarbodiimide (DIPCDI) in NMP was used and allowed to react for 45 minutes. The second coupling utilized an equimolar (to amino acid) volume of a 0.5M HBTU solution in DMF with an equal volume of a 1M DIEA solution in NMP (2:1, DIEA:amino acid) for a period of 30 minutes.

Upon completion of the synthesis, the resin was treated with MeOH, dried under reduced pressure, and then cleaved using a cocktail composed of trifluoroacetic acid (TFA) : thioanisole : ethanedithiol (EDT) : water, at a ratio of 36:2:1:1 (with the volume dependent on the amount of resin) for 2 hours (a minimum of 2 hours was used with an additional 30 minutes added for each arginine, but not exceeding 3 hours) with the first 15 minutes occurring in a wet ice bath. The solutions were then dissolved in a 10% TFA in water solution, washed 3 times with methyl t-butyl ether (MTBE) and lyophilized.

The amino termini of selected peptide-based constructs may be derivatized with acetic anhydride or other organic carboxylic acid after synthesis on solid phase using an N-terminal Fmoc protection strategy as described above. Subsequent to Fmoc removal with piperidine and prior to peptide cleavage with TFA, peptide on the resin could be derivatized with a 10-fold molar excess of acetic anhydride or 4-fold molar excess of other organic carboxylic acid with a 2-fold molar excess of diisopropylethylamine in dimethylformamide for one hour or a double coupling procedure employing N, N-diisopropylcarbodiimide (DIC) or CIPCDI/1-hydroxybenzotriazole (HOBt) and 2-(1-H-benzotriazol-1-yl)-1.1.3.3.-tetramethyluronium hexa-fluorophosphate (HBTU)/ HOBt/diisopropylethylamine (DIEA) and one of a variety of building blocks could be used for derivatization. The peptide was then cleaved from the resin with the TFA cleavage cocktail as described above and purified as described below. Derivatization, including N-terminal acetylation, of the purified peptide was verified by mass spectrometry.

Yields of the peptide-based constructs described in Table I ranged from 4.8 to 57.8%. All HPLC purities were generally greater than 90% and mass was construed by mass spectrometry.

For purity analysis of each newly synthesized peptide-based construct, dilute solutions of lyophilized peptide-based constructs were prepared and analyzed on a Michrom Ultrafast Microprotein Analyzer equipped with a 150 mm X 1 mm, 5µ particle, 300 Å pore C-8 Zorbax column. The column oven was set to 40° C, the flow rate was 100 µL/minute, and injection volumes were typically 5-10 µL. HPLC was performed using 5% acetonitrile/0.1% TFA in water as mobile phase A, and 80% acetonitrile/0.065% TFA as mobile phase B. The eluate was monitored spectrophotometrically at 214 nm. Percent purity was calculated from the peak area of the individual peptide constructs.

Selected constructs were purified by high performance liquid chromatography (HPLC), using a Waters Prep LC 2000 Preparative Chromatography System (Water Corp., Milford, MA) equipped with a Delta Pak C-18, 15 µm, 300 Å cartridge column consisting of a 40 X 10 mm guard cartridge and a 40 X 100 mm Prep Pak cartridge. The column was equilibrated in 25% buffer B, where A=5% acetonitrile/0.1% trifluoroacetic acid and B=80% acetonitrile/0.065% trifluoroacetic acid. Such peptide-based constructs were dissolved to ~ 20 mg/mL in buffer A and 200-800 mg were applied to the column through the LC pump operating at a flow rate of 8-17 mL/min. Bound material was eluted with an empirically determined gradient of buffer B, for example, a gradient of 25-35% buffer B, per 30 min applied at 8-17 mL/min. (Some constructs were purified with a gradient of 23-43% buffer B/30 min; other constructs were purified with a variety of gradients such as 25-45%, 0-60%, 30-70%, 25-75%, 30-60% or 35-75% buffer B/30 min.) The eluate was monitored at 220 and/or 280 and 300 nm with a Waters 490E Programmable Multiwavelength Detector. Fractions were collected and assayed for the construct of interest on an Ultrafast Micoprotein Analyzer (Michrom BioResources, Inc., Pleasanton, CA) equipped with a Zorbax C-8, 150 X 1 mm, 5 µm, 300 Å maintained at 40°C. Fractions containing the construct of interest at ≥90% purity were pooled and lyophilized to dryness. The purity of the recovered material was determined with analytical reverse-phase HPLC.

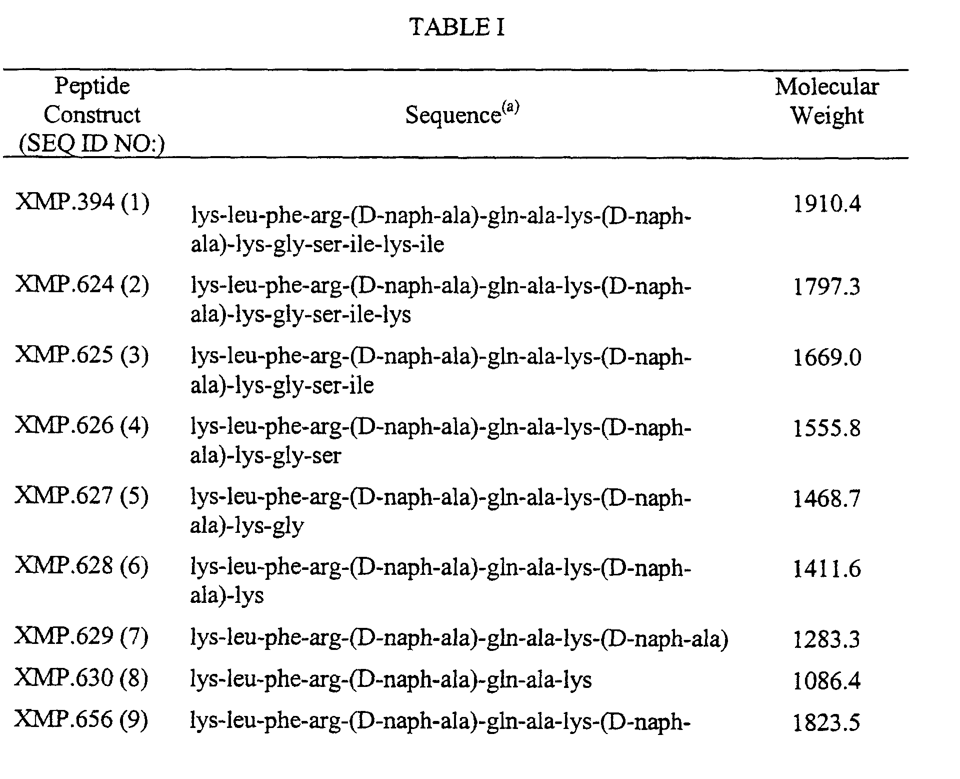

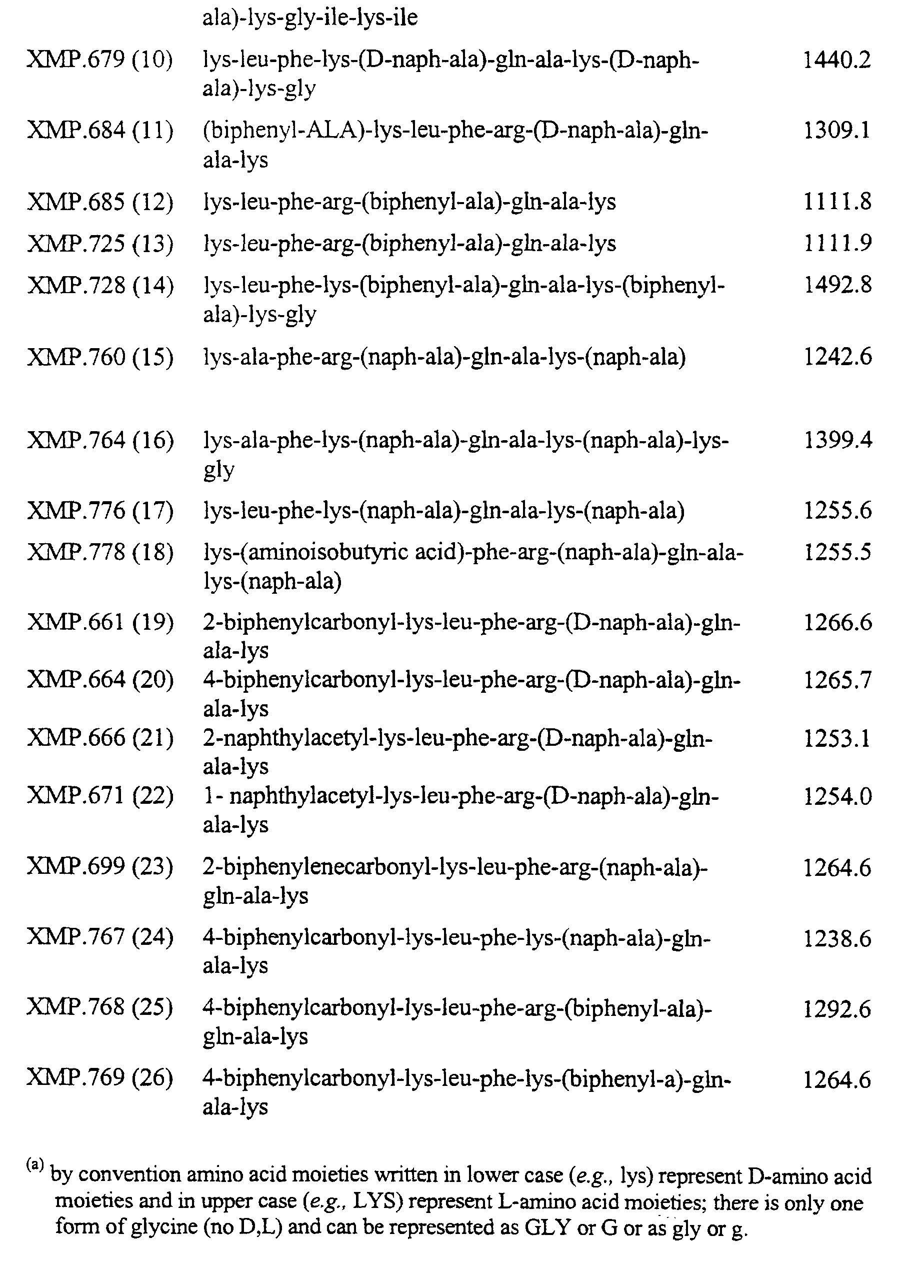

### EXAMPLE 2

### IN VITRO HUVEC ASSAY

This example addresses the activity of peptide-based constructs, including derivatized constructs, in an *in vitro* assay of endothelial cell proliferation.

Briefly, human umbilical vein endothelial cells (HUVEC) were cultured in EGM-2 medium (Clonetics Corp., San Diego, CA), harvested and plated onto a 96-well culture plate. After 24 hours of quiescence, the cells were co-cultured with various peptide-based constructs in the presence of growth factors for 72 hours to test their activity. Cell proliferation was quantitated by tritiated thymidine incorporation into DNA and was expressed in counts per minute (cpm).

Details of the experimental procedures were as follows: HUVEC cells were cultured in EGM-2 media from Clonetics Corp., a subsidiary of BioWhittaker, Inc., San Diego, CA [EGM-2 is Endothelial Cell Basal Medium-2 (EMB-2) supplemented with growth factors (hFGF-B, VEGF, IGF-1, hEGF) ascorbic acid, heparin, GA-1000 and 2% FBS), in T75 flasks until confluent (~ 4-7 days). Cells were harvested by trypsinizing the cells using 0.025% trypsin/EDTA for 5 minutes. To stop the reaction, 2-5% FBS was added. The cells were centrifuged and washed 3 times using Hank's Balanced Salt Solution (HBSS) or Dulbecco's Phosphate Buffered Saline D-PBS. The pellet was then resuspended in EBM-2 supplemented with 0.1% FBS and GA-1000 was used. The cells were counted and a cell suspension was prepared at 2 x 10⁵/mL in EBM-2 with 0.1 % FBS; cells were dispensed into a 96-well flat bottomed culture plate at 0.1 mL/well (total: ~ 20,000 cells/well in 0.1% FBS-EBM-2). Incubation continued at 37° C in 5% CO₂ for 20-24 hours. The next day peptide-based constructs were prepared for testing in a 96 well plate. The supernatant was aspirated from the cell-well, replaced with EBM-2 or the testing reagent at 20 µL/well. Then, 180 µL/well of 1.12% FBS-GFS were added to make a final concentration of 1% FBS-GFS in EBM-2. This aspiration and addition was done well-by-well. ³H-thymidine (100 µCi/mL) was added at 10 µL/well (1µCi/well, final). Incubation continued at 37° C, 5% CO₂ for 72 hours. To harvest the plate, the supernatant was aspirated off from the cell-well and then 75 µL-100 µL/well of 0.025% trypsin-EDTA was added. After incubation for 8-10 minutes at room temperature in the hood, the cells were checked and 100 µL/well of 2-5% FBS-PBS was added to stop the reaction. The plate was then harvested, dried and counted using an Inotech cell harvester (Inotech Biosystems, INB-384, Sample Processing and Filter Counting System, Lansing, MI).

Results of such HUVEC proliferation assays are shown in Table II. Tested in crude form (purity >73%) or purified, peptide-based constructs according to the invention inhibited HUVEC proliferation.

**TABLE II**

| Peptide Construct (SEQ ID NO:) | Moiety No. (Amino Acids) | Purity | IC₅₀ (µg/mL) |
|---|---|---|---|
| XMP.394 (1) | 15 | 95.2%* | 4.3 ± 0.5 |
| XMP.624 (2) | 14 | 81.5% | 5.3 ± 0.3 |
| XMP.625 (3) | 13 | 95.4% | 3.1 ± 0.2 |
| XMP.626 (4) | 12 | 92.2% | 9.1 ± 0.7 |
| XMP.627 (5) | 11 | 88.4% | 5.8 ± 0.7 |
| XMP.628 (6) | 10 | 86.0% | 6.7 ± 0.7 |
| XMP.629 (7) | 9 | 86.4% | 5.4 ± 1.2 |
| XMP.630 (8) | 8 | 73.7% | >25 |
| XMP.656 (9) | 14 | 94.1%* | 2.6 ± 0.8 |
| XMP.679 (10) | 11 | 92.8%* | 6.3 ± 0.9 |
| XMP.684 (11) | 9 | 97.8%* | 10 < × < 30 |
| XMP.685 (12) | 8 | 99.9% | ND |
| XMP.725 (13) | 8 | 87.2% | ND |
| XMP.728 (14) | 11 | 95.6% | 1 < x <3 |
| XMP.760 (15) | 9 | 98.0% | 10< x <30 |
| XMP.764 (16) | 11 | 90.0% | 10< x <30 |
| XMP.776 (17) | 9 | 96.4% | ND |
| XMP.778 (18) | 8 | 79.7% | ND |
| XMP.661 (19) | 8 | 90.5% | >30 |
| XMP.664 (20) | 7 | 99.0% | 3<×<10 |
| XMP.666 (21) | 8 | 82.9% | b |
| XMP.671 (22) | 8 | 91.0% | 10<×<30 |
| XNT.699 (23) | 8 | 75.5% | b |
| XMP.767 (24) | 8 | 96.0% | 10< x <30 |
| XMP.768 (25) | 8 | 97.2% | 3< x <10 |
| XMP.769 (26) | 8 | 98.9% | 3< x <10 |

| | | | |
|---|---|---|---|
| ND - Not Determined b - precipitated in 1 mg/ml stock solution | | | |
| * purified | | | |

Inhibitory activity with a 9-amino acid construct (XMP.629) was similar to that of the 15-amino acid parent construct (XMP.394). In repeated experiments (8 total), XMP.679 exhibited IC₅₀ values consistently in the range between 3 and 10 (3< x <10). In additional assays using purified XMP.624, XMP.625, XMP.626, XMP.627, XMP.629 and XMP.630, IC₅₀ values were comparable to those shown in Table II (4.9 ± 0.5, 1< x <3 (7 experiments), 3< x <10 (2 experiments), 3< x <10 (3 experiments), 3< x <10 and >50, respectively).

### EXAMPLE 3

### IN VIVO MATRIGEL® ANGIOGENESIS ASSAYS

This example addresses the activity of peptide-based constructs, including derivatized constructs, in *in vivo* assays of angiogenesis using Matrigel®.

For these experiments, peptide-based constructs according to the invention were assayed for their ability to inhibit heparin-induced angiogenesis *in vivo* in mice. Basement membrane matrix (Matrigel®, Becton Dickinson Labware, Mountain View, CA) was thawed and maintained at 4° C and angiogenic factors were added to the gel in the liquid state generally as described in Passaniti *et al*., 1992, *Lab*. *Invest*. 67: 519-528. Heparin sodium (Sigma, St. Louis, MO) was dissolved in sterile PBS to various concentrations ranging from 1,250 - 10,000 U/mL, and used in these experiments at 8,000 U/mL. Human recombinant basic fibroblast growth factor (bFGF; Sigma) was reconstituted into 1% BSA in PBS to 25 µg/mL. A volume of 2.5 µL dissolved heparin solution and 4.0 µL recombinant bFGF were added to 0.5 mL Matrigel® mixture per mouse injection. Peptide-based constructs were added to this Matrigel® mixture at varying concentrations ranging from 0.5 to 50 µg/mL (final concentration) in 10 µL/0.5 mL Matrigel® aliquot per experimental animal. Ten µL sterile PBS was substituted for peptide-based constructs in Matrigel® aliquots injected into control animals.

Female C57BL/6J mice (Jackson Laboratory, Bar Barbor, ME) at 6-8 weeks of age (maintained under NIH guidelines) were injected subcutaneously down the dorsal midline with 0.5 mL aliquots of Matrigel® prepared as described above. Ten days after injection, the Matrigel® gels were excised and placed in Drabkin's reagent (Sigma). Total protein and hemoglobin content can be determined for the gels stored in Drabkin's reagent after mechanical homogenization of the gels. Hemoglobin concentration was measured using Sigma Procedure #525 and reagents supplied by Sigma (St. Louis, MO) to be used with this procedure. If desired, total protein levels are determined using a microplate assay that is commercially embodied in a kit (*DC* Protein Assay, Bio-Rad, Richmond, CA).

Gels to be used for histological staining are formalin-fixed immediately after excision from the animals rather than being placed in Drabkin's reagent. Formalin-fixed gels are embedded in Tissue-Tek O.C.T. compound (Miles, Inc., Elkhart, IN) for frozen sectioning. Slides of frozen sections are stained with hematoxylin and eosin (as described by Humason, 1979, Animal Tissue Techniques, 4^{th} Ed. W.H. Feeman & Co., San Francisco, CA, Ch. 9, pp. 111-131). The effect of peptides are detected by microscopic examination of frozen stained sections for inhibition of angiogenesis relative to Matrigel® gel slices prepared without added peptides. The extent of angiogenesis inhibition is quantitated using the normalized amounts of hemoglobin found in BPI peptide-containing gel slices.

Results of Matrigel® assays with 10 µg peptide construct per gel are shown in Table III. Data are presented as the means of triplicate determinations of hemoglobin concentrations from the excised gel (ISEM) with ten mice per group.

**TABLE III**

| Peptide Construct (SEQ ID NO:) | % Inhibition | p-value |
|---|---|---|
| XMP.394 (1) | 54.6 | 0.03 |
| XMP.624 (2) | ND | ND |
| XMP.624 (3) | 51.9 | 0.04 |
| XMP.626 (4) | ND | ND |
| XMP.627 (5) | 77.6 | 0.003 |
| XMP.628 (6) | ND | ND |
| XMP.629 (7) | 30.8 | 0.22 |
| XMP.630 (8) | 49.6 | 0.05 |

| | | |
|---|---|---|
| ND - Not Determined | | |

Peptide-based constructs inhibited bFGF-induced angiogenesis. Matrigel® assay results corroborated HUVEC assay results described in Example 2.

In additional experiments, an *in vivo* angiogenesis assay was developed using Matrigel® and melanoma cells ("mel-gel" assay). Female C57B1/6J mice, as described above, were used at 6-8 weeks of age and maintained under NIH guidelines. Basement membrane matrix (Matrigel®), as described above, was thawed at 4°C prior to the addition of B16.F10 malignant melanoma cells at 50 µL per 0.5 mL of Matrlgel®. Final concentration of B16.F10 melanoma cells was varied from 10,000 to 100,000 cells/0.5 mL of Matrigel®. For this assay, peptide-based constructs at 50 µL (final concentration varies from 1.0 to 50.0 µg/0.5 mL of Matrigel®) are premixed with B16.F10 melanoma cells and added directly to Matrigel® prior to the subcutaneous injection near the abdominal midline. Fifty (50) µL sterile PBS is substituted for the constructs in Matrigel® aliquots injected into control animals. Ten days after the injections, gels are excised and placed in Drabkin's reagent (Sigma), as described above. Total hemoglobin content is determined for the gels stored in Drabkin's reagent after mechanical homogenization of the gels, as described above. Hemoglobin concentration is measured, as described above, using Sigma procedure #525 and reagents supplied by Sigma (St. Louis, MO) to be used in this procedure. Data from mel-gel assays are presented as the mean of triplicate determinations of hemoglobin concentrations from the excised gel (ISEM) with ten mice per group.

### EXAMPLE 4

### CHRONIC INFLAMMATORY DISEASE STATE MODELS: COLLAGEN-INDUCED ARTHRITIS OR REACTIVE ARTHRITIS

This example addresses the *in vivo* activity of peptide-based constructs, including derivatized constructs, when they are administered for their effects in chronic inflammatory disease states such as arthritis.

For the collagen-induced arthritis model, arthritis is induced in mice by intradermal immunization of bovine Type II collagen at the base of the tail according to the method of Stuart *et al*., 1982, *J*. *Clin. Invest.* 69: 673-683. Generally, mice begin to develop arthritic symptoms at day 21 after collagen immunization. The arthritic scores of the treated mice are then evaluated in a blinded fashion over a period of 120 days for mice treated on each of days 21-25 with injection intravenously *via* the tail vein of peptide-based constructs prepared in accordance with Example 1, or of buffer as a control.

Specifically, bovine Type II collagen (Southern Biotechnology Associates, Inc., Birmingham AL) is administered *via* intradermal injection (0.1 mg/mouse) at the base of the tail on day 0 to groups of male mice (Mouse/DBA/1J), each weighing approximately 20-25 g. Peptide-based constructs are dissolved in a buffer comprised of 0.5M NaCl, 20mM sodium acetate (pH 6.0) and diluted with PBS buffer for administration at various concentrations. PBS buffer alone (0.1 mL) is administered as a control.

The collagen-induced arthritis model is also used to evaluate the performance of peptides in comparison with protamine sulfate. Specifically, peptide-based constructs are dissolved in PBS as described above and administered at various concentrations. The other test materials are administered at the following dosages: protamine sulfate (Sigma Chemical Co., St. Louis, MO) (0.13 mg/mouse), thaumatin control protein (0.12 mg/mouse), and PBS buffer (0.1 mL). Groups of mice receive test or control materials through intravenous injection *via* the tail vein on each of days 28 through 32 post-injection with collagen.

For the reactive arthritis model, peptide-based constructs are administered to treat reactive arthritis in a *Yersinia enterocolitica* reactive arthritis model according to the method of Yong *et al*., 1988, *Microbial Pathogenesis* 4: 305-310. Specifically, peptide-based constructs are administered to DBA/2J mice which have previously been injected intravenously with *Yersinia enterocolitica* cWA 0:8 T2 (*i*.*e*., lacking the virulence plasmid according to Yong *et al*., *supra*) at a dosage of 4 x 10⁸ bacteria calculated to induce a non-septic arthritis in the mice. Groups of mice each receive test or control materials through intravenous injection via the tail vein.

*Borrelia burgdorferi* is the pathogen responsible for Lyme Disease and associated arthritis and it possesses an LPS-like complex on its cell walls which is different from but structurally related to that of *E*. *coli.* The effect of administration of the peptide-based constructs on inhibition of *B. burgdorferi* LPS in a *Limulus* Amoebocyte Lysate (LAL) inhibition assay is determined. Specifically, an LAL assay is conducted measuring the effect of peptide-based constructs on *B*. *burgdorferi* LPS administered, for example, at 2.5 µg/mL and *E*. *coli* 0113 LPS administered, for example, at 2 ng/mL.

### EXAMPLE 5

### MALIGNANT MELANOMA METASTASIS MODEL

This example addresses the activity of peptide-based constructs, including derivatized constructs in an *in vivo* malignant melanoma metastasis model.

For these experiments, peptide-based constructs, protamine, or buffer controls are administered to test their efficacy in a mouse malignant melanoma metastasis model. Specifically, groups of C57BL/6J mice are inoculated with 10⁵ B16.F10 malignant melanoma cells *via* intravenous injection into the tail vein on day 0. Peptide-based constructs prepared in accordance with Example 1 in various concentrations are administered into the tail vein of test mice on days 1, 3, 6, 8, 10, 13, 15, 17, and 19. Protamine sulfate (0.13 mg/mouse) as a positive control, or PBS buffer (0.1 mL/mouse) as a negative control are similarly administered to additional groups of control mice. The animals are either sacrificed *via* cervical dislocation on day 20 for observation of lung tissues or observed for mortality until day 40. The lobes of each lung are perfused and inflated by injecting 3 mL water into the lung *via* the trachea. Superficial tumor nodules are then counted with the aid of a dissecting microscope and the number of tumors found per group analyzed for statistically significant differences. Such analyses identify those constructs with efficacy that significantly reduce the number of tumors and significantly prolong survival.

### EXAMPLE 6

### IN VITRO ORAL ABSORPTION SCREENING

This example addresses the activity of peptide-based constructs, including derivatized constructs, in *in vitro* transport screening assays.

For these experiments, peptide-based constructs are screened for potential oral absorption in *in vitro* screening assays using MDCK and/or CACO-2 cells. Briefly, cultured monolayers of Madin-Derby canine kidney epithelial (MDCK) cells (ATCC Accession No. CCL-34) and/or CACO-2 (Human colon carcinoma) cells (Audus, K.L., et al., 1990, *Phar*. *Res.,* 7:435-451) are grown upon collagen-coated, permeable-filter supports (Becton Dickinson, Mountain View, CA). The cells are grown to confluency and allowed to differentiate (e.g., about 3 days for MDCK cells or about 21 days for CACO-2 cells). The integrity of the monolayers is determined by measuring the transepithelial resistance. The cells are incubated with a peptide-based construct on the apical side for 2.5 hours in MDCK or CACO-2 screening. The transepithelial transport of the construct is measured by quantitative HPLC analysis of the incubation media on the basolateral side of the cells (forward transport). Radiolabelled mannitol and/or cortisone are used as positive controls. In addition, the efflux of the construct is measured by quantitative HLPC analysis of the incubation media on the apical side of the cells. This follows incubation of the cells with construct on the basolateral side for 2.5 hours.

Details of the experimental procedures were as follows: MDCK or CACO-2 cells are grown to ~ 90% confluency in T75 tissue culture flasks in cell growth media (for MDCK cells, Minimum Essential Medium (Eagle's) GIBCO (Grand Island, NY) #11095-080 or for CACO-2 cells, DMEM, GIBCO #11965-050, high glucose - 500 mL; FBS 10% (Hyclone) heat treated - 50 mL; L-Glutamine (200 mM) GIBCO #25030-016 - 5 mL; for CACO-2 cells, Non-essential amino acids, GIBCO #11140-050 (200 mM, 100x) - 5.5 mL; and Penicillin/Streptomycin (100 µg/mL) GIBCO #15140-015 - 5mL, that has been filtered and stored at 4° C). The cells are trypsinized (- 20 minutes for MDCK cells for passaging due to their tight adherence) and seeded on 24.5 mm transwells (Transwell-COL, #3245, Corning CoStar Corp., Cambridge, MA) at a concentration of ∼ 3 x 10⁶ MDCK cells/well or ~ 3 x 10⁵ CACO-2 cells/well. For the MDCK cells, media is changed one day post seeding and the MDCK cells are allowed to grow for an additional 2 days. For the CACO-2 cells, media is changed every 2 days post seeding and the CACO-2 cells are allowed to grow for a total of up to 21 days.

Following the days post seeding, the cells are ready for transport experiments. The cells are fed fresh media 2 hours before starting the transport experiment. The cells are then washed with transport media (TM: Hank's Balanced Salt Solution (HBSS) GIBCO #14025-027, with no phenol red; 10 mM HEPES (from 1 M HEPES) GIBCO #15630-015, pH balanced with NaOH to 7.4) and placed in new 6 well plates. Donor solutions of peptide-based constructs for assay are prepared in 1.5 mL TM at a concentration of ∼ 100 µg/mL. For forward transport studies, these 1.5 mL construct solutions are added to the apical chamber of the transwells. Approximately 2.6 mL of acceptor solution (TM only, no construct) is added to the basolateral chamber. For efflux studies, the donor solution with construct is added to the basolateral chamber and acceptor solution (TM only, no construct) is added to the apical chamber. Each construct is assayed at least in triplicate.

The transwells are returned to the 37° C tissue culture incubator for 2.5 hours. At the end of 2.5 hours the apical and basolateral solutions are separately freeze dried (lyophilized) in high vacuum in 15 mL conical tubes. The samples are then resuspended in 200 µL HPLC buffer A (5% acetonitrile: 95% water: 0.1% tetrafluoroacetic acid (TFA)) and 50 µL of the resuspended sample is used for HPLC analysis.

Apical control wells containing 1.5 mL TM with tritiated mannitol (³H-mannitol, 1µCi/mL; Dupont NEN Research Products, #NET-101, Boston, MA) are analyzed separately for counts per minute (cpm) of tritium to test the integrity of the MDCK cell monolayers.

Forward transport is calculated as the percentage of peptide-based construct in the basolateral chamber (determined by the area under the HPLC peak) to the initial concentration of the apical solution (initial donor concentration of ~ 100 µg/mL). For efflux experiments, the reverse calculation is made (percentage of peptide-based construct in the apical chamber to the initial construct concentration in the basolateral solution). Such intestinal absorption screening identifies constructs that are potential orally available compounds.

### EXAMPLE 7

### IN VIVO ORAL ABSORPTION SCREENING

This example addresses the activity of peptide-based constructs, including derivatized constructs, for oral absorption in an *in vivo* screening assay in which constructs are administered by oral gavage to mice.

Briefly, serum concentrations of the constructs are measured at various time intervals after administration by HPLC. For example, constructs may be administered to mice at various dosages (*e*.*g*., 10 mg/kg body weight or 20 mg/kg body weight) and serum peptide concentrations are measured at various time intervals (*e.g*., 1 hours, 4 hours, and/or 24 hours) after administration to the mice. HPLC analysis identifies constructs that are absorbed after oral administration. Such constructs showing increased oral availability may achieve therapeutically effective serum concentrations after oral administration.

### EXAMPLE 8

### ORAL ACTIVITY

This example addresses the activity of peptide-based constructs, including derivatized constructs, for activity upon oral administration (oral activity) in an *in vivo* animal model.

Animal models useful for testing oral activity include those described in Examples 4 and 5 above. Treatment is initiated by oral gavage (~ 400 µl) of either 0.5% dextrose, or the test peptide-based construct in 0.5% dextrose at various dosage levels (*e*.*g*., 10 mg/kg or 20 mg/kg according to the dosing regimen). Daily monitoring for efficacy is performed. The animals treated with orally active peptide-based constructs show improvement compared with the dextrose-treated controls.

### EXAMPLE 9

### RETINAL NEOVASCULARIZATION MODEL

This example addresses the *in vivo* activity of peptide-based constructs, including derivatized constructs, when they are administered for their effects in a retinal neovascularization model.

In these experiments, peptide-based constructs are tested for their activity on the action of VEGF, bFGF, IGF-1 and conditioned media (hypoxia and hyperglycemia) on the *in vitro* growth and migration of retinal pigmented epithelial cells (RPE), retinal microvessel pericytes, and retinal endothelial cells. Effects are evaluated on gene expression under hypoxic or hypoglycemic conditions. In additional experiments, the effects of such constructs are characterized *in vivo* on the neovascular responses to hyperoxia in the retina of the neonatal mouse.

For the *in vitro* culture experiments, retinal microvessel pericytes, retinal endothelial cells, and retinal pigmented epithelial cells (RPE) are cultured from bovine retina. Dose responses and time course for growth and migration as stimulated by VEGF, bFGF, and IGF-1 are performed. For example, retinal endothelial cell growth as stimulated by VEGF was inhibited with 5 µg/mL and 15 µg/mL XMP.679 as measured by DNA content (ng/cell well). The cell growth as measured by DNA content in the XMP.679-treated VEGF-stimulated cells was comparable with that of cells not stimulated by VEGF. In addition, conditioned media collected from cultured RPE, pericytes, and endothelial cells exposed to hypoxic or hyperglycemic conditions is collected in serum free media. When the dose responses of these growth factors and conditioned media are determined, the effect of peptide-based constructs independently, and in combination with the growth factors described above, are tested in growth and migration assays.

For the *in vivo* experiments, studies are performed to evaluate the effect of peptide-based constructs on retinal neovascularization. Neonatal mice are exposed to 100% O₂ for an extended period of time and then removed. This increased oxygenation delays development of the retinal vasculature such that, when they are returned to room air, the retinas are severely hypoxic and release of many cytokines occur, including VEGF, bFGF, IGF-1, and others. Retinal neovascularization occurs in 100% of animals. The severity of retinal neovascularization is quantitated by counting endothelial cell nuclei internal to the internal limiting membrane in retinal sections. VEGF and KDR expressions are evaluated by *in situ* hybridization, Northern and Western blot analysis. Tyrosine phosphorylation pattern of the whole retina is also evaluated by Western blot analysis. Peptide-based constructs are identified that inhibit or reduce retinal neovascularization.

### EXAMPLE 10

### HEPARIN BINDING AND NEUTRALIZATION

This example addresses heparin binding and heparin neutralizing activities of peptide-based constructs, including derivatized constructs.

A Chromostrate™ assay was used to determine the effect of peptide-based constructs, including derivatized constructs, and the effect protamine sulfate (as a positive control) on Factor Xa neutralization by ATIII/heparin complexes. The assay was conducted using a Chromostrate™ heparin anti-Factor Xa assay kit (Organon Teknika Corp., Durham, N.C.). Heparin concentration was varied so that a heparin standard curve was generated. The assay measured the potentiating effect of heparin on anti-Xa activity *in vitro.* Solution containing heparin is incubated in the presence of ATIII with an excess of Factor Xa, forming an ATIII-Heparin-Xa complex. The remaining Xa catalyzes the release of p-nitroaniline (pNA) from the chromogenic substrate. The release of p-nitroaniline is measured at 405 nm. The absorbance obtained in inversely porportional to the concentration of heparin in the sample.

Reagents for the assay are prepared as follows. Heparin, peptide-based constructs and protamine sulfate stocks are made in saline. Stock solutions are approximately 1mg/mL=1µg/µL. Factor Xa reagent and substrate reagent are reconstituted with 2.0 mL purified water. Antithrombin III (ATIII) reagent is reconstituted with 1.0 mL purified water. The assay is performed in 96 well microtiter plates. Assay components are added to the microtiter wells as follows: 12.5 µl of heparin (from 0 to 10 U/mL), 25 µl of construct or protamine sulfate, 12.5 µL of ATIII (0.5 PEU/mL), 25 µL of bovine Factor Xa (14 nKat/mL), 25 µL substrate (3 µmoles/mL). The reaction is allowed to proceed for 20 minutes and then 25 µL of 0.1M acetic acid is added. The color reaction is quantitated on a microplate reader at 405nm. Constructs that neutralize heparin show an increase in the absorbance at 405 nm. Protamine is used as a positive control and its activity is designated as 100%. The percent (%) heparin neutralization for each peptide construct is calculated relative to the neutralization by protamine (100%), and shown in Table IV.

**TABLE IV**

| Peptide Construct (SEQ ID NO:) | % Assay A^{a} | % Assay B^{a} |
|---|---|---|
| XMP.394 (1) | 143% | 133% |
| XMP.624 (2) | 131% | 94% |
| XMP.625 (3) | 79% | 125% |
| XMP.626 (4) | 99% | 80% |
| XMP.627 (5) | 98% | 62% |
| XMP.628 (6) | 107% | 68% |
| XMP.629 (7) | 69% | 49% |
| XMP.630 (8) | 2.3% | 0.5% |
| XMP.656 (9) | 74% | 65% |
| XMP.679 (10) | 96% | 94% |
| XMP.684 (11) | ND | ND |
| XMP.685 (12) | 3.4% | 0.2% |
| XMP.725 (13) | 3.8% | 1.0% |
| XMP.728 (14) | 106% | 87% |
| XMP.760 (15) | 53% | 44% |
| XMP.764 (16) | 75% | 60% |
| XMP.776 (17) | 74% | 75% |
| XNP.778 (18) | 54% | 49% |
| XMP.661 (19) | 30% | 9% |
| XNP.664 (20) | 50% | 16% |
| XMP.666 (21) | 39% | 18% |
| XMP.671 (22) | 32% | 15% |
| XMP.699 (23) | 45% | 23% |
| XMP.767 (24) | 63%* | 58% |
| XMP.768 (25) | 34% | 10% |
| XMP.769 (26) | 64% | 46% |

| | | |
|---|---|---|
| ^{a} Protamine control was 100% | | |
| * from a repeated experiment | | |
| ND - Not Determined | | |

### EXAMPLE 11

### ADDITIONAL ACTIVITY ASSAYS

This example addresses the testing of peptide-based constructs, including derivatized constructs, for their activity, in a variety of assays known in the art for activities of BPI protein products.

Assays useful for testing a variety of biological activities of BPI protein products, including BPI-derived peptides, are described in co-owned U.S. Patent Nos. 5,733,872; 5,763,567; 5,652,332; and 5,856,438 and corresponding PCT Publication Nos. WO 94/20532 (PCT/US94/02465) and WO 95/19372 (PCT/US94/10427) and U.S. Patent No. 5,858,974 and corresponding PCT Publications Nos. WO 96/08509 (PCT/US95/09262) and WO 97/04008 (PCT/US96/03845), incorporated herein by reference. These activities include antimicrobial (including antibacterial and antifungal), LPS binding, LPS neutralization and additional assays of heparin binding as well as heparin neutralization. When the peptide-based constructs, including the derivatized constructs, shown in Table I were tested in broth and/or radial diffusion assays (substantially in accordance with for example, Example 2 of U.S. Patent No. 5,858,974 except that for the broth assays with *Candida* cells were diluted to 5x10³ cells/mL instead of 2x10⁶ cells/mL in broth, and Examples 2 and 13 of U.S. Patent No. 5,652,332 except that for the broth assays with the bacteria tryptic soy broth was used instead of Mueller Hinton) for their antimicrobial effect on *E*. *coli* (J5 and 0111B4), *Staphylococcus aureus* and *Candida albicans*, each construct showed antimicrobial activity against one or more bacterial strains and/or against *Candida.* The non-derivatized constructs with the highest antimicrobial activities as tested included XMP.627, XMP.628, XMP.629, XMP.656, XMP.679, XMP.760, XMP.764, XMP.776 and XMP.778. The derivatized constructs with the highest activities as tested included XMP.664 and XMP.767.

In other assays of LPS neutralization, exemplary constructs of the invention such as XMP.624, XMP.625, XMP.626, XMP 628 and XMP.629 showed activity in a RAW cell assay (substantially in accordance with for example, Example 7 of U.S. Patent No. 5,858,974, and Example 20D of U.S. Patent No. 5,652,332; other LPS assays include Examples 4, 20A, B, C, E, F and G, 25 and 26 of U.S: Patent No. 5,652,332).

Additional heparin binding and heparin neutralizing assays both *in vitro* and *in vivo* (including for example, Examples 6, 11, 17 and 21 of U.S. Patent No. 5,652,332), have been described for BPI protein products and are useful for testing peptide-based constructs of the invention.

Other peptide-based constructs are described in U.S. Application No. 09/344,541 and the concurrently filed corresponding PCT Application No. PCT/US00/17383(WO 01/00671).

## Claims

1. A composition of 8-15 amino acid moieties consecutively linked by peptide bonds, said composition having at least one or more of heparin binding, heparin neutralizing, endothelial cell proliferation inhibiting, antiangiogenic, LPS binding, LPS neutralizing or antimicrobial properties and comprising a sequence of the formula:
KLFR(naph-A)QAR₃ ,
wherein the symbols may refer both to D- or to L-amino acid residue,
wherein R₃ is any one of K, K(naph-A), K(naph-A)K, K(naph-A)KG, K(naph-A)KGS, K(naph-A)KGSI, K(naph-A)KGSIK or K(naph-A)KGSIKI; or
wherein the carboxyl terminal group is amidated or nonamidated,
and optionally, at least one conservative substitution of amino acid moieties;
wherein the composition is not k-l-f-r (naph-a)-q-a-k-(naph-a)-k-g-s-i-k-i (SEQ ID NO: 1).

2. The composition of claim 1 comprising two or more conservative substitutions of amino acid moieties.

3. A peptide-based construct or composition of amino acid moieties consecutively linked by peptide bonds selected from:

4. A composition of 8-15 amino acid moieties consecutively linked by peptide bonds, said composition having at least one of heparin binding, heparin neutralizing, endothelial cell proliferation inhibiting, antiangiogenic, LPS binding, LPS neutralizing or antimicrobial properties and comprising a sequence of the formula:
R₁KLFR(naph-A)QAR₃
wherein R₁ is any one of R₂-CH₂-, R₂-CH₂-CO-, R₂-CO-, R₂-SO_{y}-, or R₂-PO_{z}-;
wherein,
y=0-3,
z=1-4;
R₂ is a hydrophobic moiety that is any one of a cyclic molecule having at least 3 carbon atoms, a heterocyclic molecule having at least 3 atoms, a functionalized cyclic molecule having at least 3 carbon atoms, or a functionalized heterocyclic molecule having at least 3 atoms;
wherein R₃ is any one of K, K(naph-A), K(naph-A)K, K(naph-A)KG, K(naph-A)KGS, K(naph-A)KGSI, K(naph-A)KGSIK or K(naph-A)KGSIKI;
wherein the carboxyl terminal group is amidated or nonamidated,
and, optionally, comprising at least one conservative substitution of amino acid moieties;
wherein the composition is not k-l-f-r (naph-a)-q-a-k-(naph-a)-k-g-s-i-k-i (SEQ ID NO:1).

5. The composition of claim 4 comprising two or more conservative substitutions of amino acid moieties.

6. The composition of claim 4, wherein said R₂ is a hydrophobic moiety that is any one of (a) an optionally substituted carbocyclic ring, saturated or partially or fully unsaturated containing 3 to 8, preferably 5 or 6, carbon atoms; (b) an optionally substituted heterocyclic ring, saturated or partially or fully unsaturated, containing 3 to 8, preferably 5 or 6, atoms, wherein at least one atom is a heteroatom that is any one of oxygen, nitrogen, or sulphur; or (c) an optionally substituted bicyclic ring 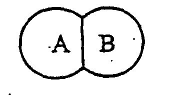 wherein the fused rings A and B, independently, are a 5- or 6-membered ring, saturated or partially or fully unsaturated, and comprise carbon atoms and optionally one to three heteroatoms selected from oxygen, sulphur, or nitrogen; wherein if there is more than one heteroatom, each may be the same or different.

7. The composition of claim 4, wherein said R₂ is a hydrophobic moiety that is any one of biotin, 2-biphenylene, 2-anthraquinone, 2-benzofuran, 2-indole, 1-isoquinoline, hydroxyphenyl, 2-quinoline, 1-[3-(3,4-dihydroxycinnamoyl)-1,3,4,5-tetrahydroxycyclohexyl], 1-(3,5-dichloro-2-hydroxyphenyl), 1-(3,5-diiodo-2-hydroxyphenyl, 1-(3,5-dinitro-2-hydroxyphenyl), 1-(4-azido-2-hydroxyphenyl), 4-biphenyl, 2-biphenyl, 1-naphthyl, 2-naphthyl, 3-amino-2-naphthyl, 3-chloro-2-nitrophenyl, 3,4-dihydroxyphenyl, 3,4,5-trihydroxyphenyl, 2-chloro-3-nitrophenyl, 5-azido-2-nitrophenyl, 3-amino-2-pyrazyl, 2-benzyloxycarbonyl-ethyl, 2-thienyl, 2-(3,4-dihydroxyphenyl)ethylene, 5-bromo-3-indolemethylene, 2-(4-hydroxy-3-methoxyphenyl)ethylene, 2-(3-chlorophenyl)ethylene, 2-pyrazyl, 4-imidazolyl, 2-imino-1-imidazolidyl, pyridyl, 3-piperidyl, 4-piperidyl, fluorescein, 2-(4-amino-3,5,6-trichloro-pyridy 1), 3-(2-chloro-6-fluorophenyl)-5-methylisoxazolyl, or 4-azido-phenyl.

8. A peptide-based construct or composition of amino acid moieties consecutively linked by peptide bonds selected from: or

9. A peptide-based construct or composition of 8-15 amino acid moieties in length having a sequence which is a subsequence of functional Domain II (amino acids 65-99) of bactericidal/ permeability -increasing (BPI) protein in reverse order, wherein the peptide-based construct or composition is not k-l-f-r (naph-a)-q-a-k-(naph-a)-k-g-s-i-k-i (SEQ ID NO: 1).

10. A derivatized peptide-based construct or composition of 8-15 amino acid moieties in length having a sequence which is a subsequence of functional Domain II (amino acids 65-99) of bactericidal/ permeability - increasing (BPI) protein in reverse order, said sequence being covalently linked to a hydrophobic moiety, wherein the peptide-based construct or composition is not k-l-f-r (naph-a)-q-a-k (naph-a)-k-g-s-i-k-i (SEQ ID NO: 1).

11. A construct or composition of claim 10, wherein the hydrophobic moiety is covalently linked to the N-terminus of the sequence.

12. A construct or composition of claim 10 or 11, wherein the hydrophobic moiety is R₂, wherein R₂ is any one of a cyclic molecule having at least 3 carbon atoms, a heterocyclic molecule having at least 3 atoms, a functionalized cyclic molecule having at least 3 carbon atoms, or a functionalized heterocyclic molecule having at least 3 atoms.

13. A construct or composition of any one of claims 10 to 12, wherein the reverse subsequence is amino acids 99-92, 99-91, 99-90, 99-89, 99-88, 99-87, 99-86, or 99-85 of BPI protein, and wherein the reverse subsequence is substituted at positions 95 and 91.

14. A construct or composition according to any one of claims 1 to 13 having heparin neutralizing properties.

15. A construct or composition according to any one of claims 1 to 13 having endothelial cell proliferation inhibiting properties.

16. A construct or composition according to any one of claims 1 to 13 having antiangiogenic properties.

17. A construct or composition according to any one of claims 1 to 16, wherein the sequence contains D-amino acid moieties.

18. A construct or composition according to any one of claims 1 to 17, wherein the first two amino-terminal amino acid moieties are D-amino acid moieties and the last two carboxy-terminal amino acid moieties are D-amino acid moieties.

19. A pharmaceutical composition comprising a construct or composition according to any one of claims 1 to 18 and a pharmaceutically acceptable adjuvant, diluent, or carrier.

20. A construct or composition according to any one of claims 1 to 18 for use in therapy.

21. Use of a construct or composition according to any one of claims 1 to 19 for the manufacture of a medicament for binding and/or neutralizing an exogenous or therapeutically administered heparin compound, for binding and/or neutralizing heparin, or for treating a heparin-related or heparin-mediated disorder, condition or disease, or for the manufacture of a medicament for antimicrobial activity, for binding and/or neutralising LPS, or for treating an infection or a disorder associated with endotoxin.

22. The use of claim 21, wherein the medicament is for concurrent administration with another therapeutic agent.

23. The use of claim 22, wherein the medicament is for administration before or after the other therapeutic agent.

24. The use of a construct or composition according to any one of claims 1 to 19 in combination with another therapeutic agent for the manufacture of a medicament for binding and/or neutralizing an exogenous or therapeutically administered heparin compound, for binding and/or neutralizing heparin, or for treating a heparin-related or heparin-mediated disorder, condition or disease, or for the manufacture of a medicament for antimicrobial activity, for binding and/or neutralizing LPS, or for treating an infection or a disorder associated with endotoxin.

25. The use according to any one of claims 21 to 24, wherein the medicament is for neutralizing the anticoagulant effect of an exogenous heparin compound administered to a mammal.

26. The use of claim 25, wherein the clotting time of said mammal is returned to normal.

27. A use according to any one of claims 21 to 26, wherein the exogenous heparin compound has been administered during cardiopulmonary bypass surgery, cardiac catheterization or angioplasty, hemodialysis, or to a patient at risk of or suffering from thrombosis, including deep venous thrombosis, acute myocardial infarction, stroke or pulmonary embolism.

28. The use of any one of claims 21 to 24, wherein the medicament is for treating a disorder associated with endothelial cell proliferation.

29. The use of any one of claims 21 to 24, wherein the medicament is for inhibiting endothelial cell proliferation in a mammal.

30. The use of any one of claims 21 to 24, wherein the medicament is for inhibiting angiogenesis in a mammal.

31. The use of claim 30, wherein said angiogenesis is in the eye.

32. A use according to claim 31, wherein the angiogenesis in the eye is involved in ocular neovascularisation, proliferative retinopathy, retrolental fibraplasia, macular degeneration, neovascular glaucoma or diabetic ocular disease, including diabetic iris neovascularization or retinopathy.

33. The use of any one of claims 21 to 24, wherein the medicament is for treating a mammal suffering from a disorder, condition or disease associated with or involving angiogenesis.

34. The use of claim 33, wherein the medicament is for inhibiting progression of the disorder, condition or disease, or for ameliorating signs and symptoms of the disorder, condition or disease.

35. The use of claim 33, wherein said disorder involving angiogenesis is a chronic inflammatory disease.

36. The use of claim 35, wherein said chronic inflammatory disease is chronic pancreatitis, dermatosis associated with chronic inflammation, including psoriasis, cirrhosis, asthma, multiple sclerosis, arthritis, including rheumatoid arthritis, reactive arthritis or chronic inflammatory arthritis, autoimmune disorders, including vasculitis, glomerulonephritis, experimental allergic encephalomyelitis (EAE), lupus, myasthenia gravis, ulcerative colitis, Crohn's disease, inflammatory bowel disease, chronic inflammation associated with hemodialysis, granulocyte transfusion associated syndrome; rejection reactions after allograft or xenograft transplanation, including graft versus host disease; or other chronic inflammatory disorders.

37. The use of claim 33, wherein said disorder involving angiogenesis is growth, proliferation or metastasis of tumour cells.

38. A use according to Claim 37, wherein the medicament is for promoting tumour regression in adult or pediatric oncology, including reducing growth of solid tumours/malignancies, locally advanced tumours, metastatic cancer, human soft tissue sarcomas, cancer metastases, including lymphatic metastases, blood cell malignancies, effusion lymphomas (body cavity based lymphomas), lung cancer, including small cell carcinoma, non-small cell cancers, breast cancer, including small cell carcinoma or ductal carcinoma, gastrointestinal cancers, including stomach cancer, colon cancer, colorectal cancer, polyps associated with colorectal neoplasia, pancreatic cancer, liver cancer, urological cancers, including bladder cancer, prostate cancer, malignancies of the female genital tract, including ovarian carcinoma, uterine endometrial cancers, or solid tumours in the ovarian follicle, kidney cancer, including renal cell carcinoma, brain cancer, including intrinsic brain tumours, neuroblastoma, astrocytic brain tumours, gliomas, metastatic tumour cell invasion in the central nervous system, bone cancers, including osteomas, skin cancers, including malignant melanoma, tumour progression of human skin keratinocytes, or squamous cell cancer, hemangiopericytoma, or Kaposi's sarcoma.

39. A use according to Claim 33, wherein the medicament is for the treatment of atherosclerosis, ischemic heart disease, myocardial infarction, coronary heart disease, restenosis, including restenosis following balloon angiography, neointimal hyperplasia, disruption of intercellular junctions in vascular endothelium, hypertension, vessel injury, arterial ischemia, arterial stenosis, peripheral vascular disease or stroke.

40. A use according to any one of claims 21 to 39, wherein the medicament is for systemic or topical administration.

41. The use of claim 40, wherein the medicament is for oral, intravenous, intramusclar or subcutaneous, intraocular and retrobulbar, intrathecal, intraperitoneal, intrapulmonary, or transdermal administration.

42. The use of claim 40, wherein the medicament is for administration in the form of salves, ophthalmic drops, ear drops, or irrigation fluids.

43. A use according to any of claims 1 to 40, wherein the medicament is for parenteral administration in a dose ranging from 1 µg/kg to 100mg/kg per day.

44. An *in vitro* method of neutralizing the anticoagulant effect of heparin comprising contacting the heparin with a construct or composition according to any one of claims 1 to 18.

45. A method for identifying a derivatized peptide sequence derived from or based on the sequence identified and selected from Domain II of bactericidal/permeability-increasing protein (BPI) having biological activity and epithelial absorption of at least 0.001 % comprising the steps of:
(a) derivatizing a peptide sequence based on a sequence, subsequence, reverse sequence or reverse subsequence of Domain II of BPI through covalent linkage of a hydrophobic moiety or moieties at the N-terminus, C-terminus or within said peptide sequence;
(b) measuring the activity of said derivatized peptide sequence obtained in step (a) wherein the activity is any one or more of heparin binding, heparin neutralizing, endothelial cell proliferation inhibiting, antiangiogenic, LPS binding, LPS neutralizing or antimicrobial properties and
(c) measuring the epithelial absorption of said derivatized peptide sequence obtained in step (a).

46. A method for designing and identifying a biologically active derivatized peptide-based sequence, prophylactic or therapeutic medicament derived from or based on the peptide sequence identified and selected from BPI or a fragment thereof with epithelial absorption of at least 0.001%, said method comprising the steps of:
(a) identifying a target peptide sequence derived from or based on the polypeptide sequence of BPI or a fragment thereof which exhibits activity *in vitro* or *in vivo* wherein the activity is any one or more of heparin binding, heparin neutralizing, endothelial cell proliferation inhibiting, antiangiogenic, LPS binding, LPS neutralizing or antimicrobial properties;
(b) constructing a library of minimum length, activity retaining peptide sequences (MinLARPS) by substituting or deleting amino acid moieties within said target peptide sequence;
(c) measuring the activity of said MinLARPS to determine the minimum number of residues necessary to retain activity of at least 1% of that of said target polypeptide sequence wherein the activity is any one or more of heparin binding, heparin neutralizing, endothelial cell proliferation inhibiting, antiangiogenic, LPS binding, LPS neutralizing or antimicrobial properties;
(d) measuring epithelial absorption of said MinLARPS in *in vitro* assays to identify which of said MinLARPS retain epithelial absorption of at least 0.001%;
(e) synthesizing derivatized MinLARPS by chemically modifying said MinLARPS through covalent linkage of a hydrophobic moiety or moieties linked at the N-terminus, C-terminus, or within the sequence of said MinLARPS;
(f) repeating steps (c) and (d) with said derivatized MinLARPS.

## Patentansprüche

1. Eine Zusammensetzung aus 8-15 Aminosäureeinheiten, die aufeinanderfolgend über Peptidbindungen verknüpft sind, wobei die Zusammensetzung wenigstens eine oder mehrere Eigenschaften hat aus: Heparinbindung, Heparinneutralisierung, Inhibition der Endothelzellproliferation, anti-angiogene Eigenschaft, LPS-Bindung, LPS-Neutralisierung oder antimikrobielle Eigenschaften, und eine Sequenz der Formel umfaßt:
KLFR(naph-A)QAR₃,
wobei die Symbole sich sowohl auf D- oder L-Aminosäurereste beziehen können,
wobei R₃ eines ist aus K, K(naph-A), K(naph-A)K, K(naph-A)KG, K(naph-A)KGS, K(naph-A)KGSI, K(naph-A)KGSIK oder K(naph-A)KGSIKI; oder
wobei die carboxy-terminale Gruppe amidiert oder nicht-amidiert ist,
und fakultativ wenigstens eine konservative Substitution der Aminosäureeinheiten umfaßt;
wobei die Zusammensetzung nicht k-l-f-r (naph-a)-q-a-k-(naph-a)-k-g-s-i-k-i ist (SEQ ID NO: 1).

2. Zusammensetzung nach Anspruch 1, umfassend zwei oder mehrere konservative Substitutionen von Aminosäureeinheiten.

3. Ein Konstrukt auf Peptid-Basis oder eine Zusammensetzung von Aminosäureeinheiten, die aufeinanderfolgend durch Peptidbindungen verknüpft sind, ausgewählt aus: oder

4. Eine Zusammensetzung aus 8-15 Aminosäureeinheiten, die aufeinanderfolgend durch Peptidbindungen verknüpft sind, wobei die Zusammensetzung wenigstens eine Eigenschaft hat aus: Heparinbindung, Heparinneutralisierung, Inhibition der Endothelzellproliferation, anti-angiogene Eigenschaft, LPS-Bindung, LPS-Neutralisierung oder antimikrobielle Eigenschaften, und eine Sequenz der Formel umfaßt:
R₁KLFR(naph-A)QAR₃,
wobei R₁ eines ist von R₂-CH₂-, R₂-CH₂-CO-, R₂-CO-, R₂-SO_{y}- oder R₂-PO_{z}-;
wobei
y=0-3,
z=1-4;
R₂ eine hydrophobe Gruppe ist, die irgendein beliebiges Molekül ist aus: ein zyklisches Molekül mit wenigstens 3 Kohlenstoffatomen, ein heterozyklisches Molekül mit wenigstens 3 Atomen, ein funktionalisiertes zyklisches Molekül mit wenigstens 3 Kohlenstoffatomen oder ein funktionalisiertes heterozyklisches Molekül mit wenigstens 3 Atomen;
wobei R₃ ein beliebiges ist aus: K, K(naph-A), K(naph-A)K, K(naph-A)KG, K(naph-A)KGS, K(naph-A)KGSI, K(naph-A)KGSIK oder K(naph-A)KGSIKI;
wobei die carboxy-terminale Gruppe amidiert oder nicht-amidiert ist;
und fakultativ wenigstens eine konservative Substitution der Aminosäureeinheiten umfaßt;
wobei die Zusammensetzung nicht k-l-f-r (naph-a)-q-a-k-(naph-a)-k-g-s-i-k-i ist (SEQ ID NO: 1).

5. Zusammensetzung nach Anspruch 4, umfassend zwei oder mehrere konservative Substitutionen von Aminosäureeinheiten.

6. Zusammensetzung nach Anspruch 4, wobei R₂ eine hydrophobe Einheit ist, die ein beliebiges ist von: (a) ein fakultativ substituierter Kohlenstoff-zyklischer Ring, gesättigt oder teilweise oder vollständig ungesättigt, enthaltend 3 bis 8, bevorzugt 5 oder 6 Kohlenstoffatome;
(b) ein fakultativ substituierter heterozyklischer Ring, gesättigt oder teilweise oder vollständig ungesättigt, enthaltend 3 bis 8, bevorzugt 5 oder 6 Atome, wobei wenigstens ein Atom ein Heteroatom ist, das eines ist aus Sauerstoff, Stickstoff oder Schwefel; oder (c) ein fakultativ substituierter bizyklischer Ring 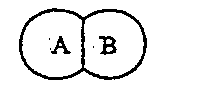
wobei die kondensierten Ringe A und B unabhängig voneinander ein 5- oder 6-gliedriger Ring sind, gesättigt oder teilweise oder vollständig ungesättigt, und Kohlenstoffatome und fakultativ ein bis drei Heteroatome umfassen, ausgewählt aus Sauerstoff, Schwefel oder Stickstoff;
wobei, wenn mehr als ein Heteroatom vorhanden ist, alle dieselben oder unterschiedlich sein können.

7. Zusammensetzung nach Anspruch 4, wobei R₂ eine hydrophobe Einheit ist, die eine beliebige ist aus: Biotin, 2-Biphenylen, 2-Anthrachinon, 2-Benzofuran, 2-Indol, 1-Isochinolin, Hydroxyphenyl, 2-Chinolin, 1-[3-(3,4-Dihydroxycinnamoyl)-1,3,4,5-tetrahydroxycyclohexyl], 1-(3,5-Dichlor-2-hydroxyphenyl), 1-(3,5-Diiod-2-hydroxyphenyl), 1-(3,5-Dinitro-2-hydroxyphenyl), 1-(4-Azido-2-hydroxyphenyl), 4-Biphenyl, 2-Biphenyl, 1-Naphthyl, 2-Naphthyl, 3-Amino-2-naphthyl, 3-Chlor-2-Nitrophenyl, 3,4-Dihydroxyphenyl, 3,4,5-Trihydroxyphenyl, 2-Chlor-3-nitrophenyl, 5-Azido-2-nitrophenyl, 3-Amino-2-pyrazyl, 2-Benzyloxycarbonyl-ethyl, 2-Thienyl, 2-(3,4-Dihydroxyphenyl)ethylen, 5-Brom-3-indolmethylen, 2-(4-Hydroxy-3-methoxyphenyl)ethylen, 2-(3-Chlorphenyl)ethylen, 2-Pyrazyl, 4-Imidazolyl, 2-Imino-1-imidazolidyl, Pyridyl, 3-Piperidyl, 4-Piperidyl, Fluorescein, 2-(4-Amino-3,5,6-trichlorpyridyl), 3-(2-Chlor-6-fluorphenyl)-5-methylisoxazolyl oder 4-Azido-phenyl.

8. Ein Konstrukt auf Peptid-Basis oder eine Zusammensetzung von Aminosäureeinheiten, die aufeinanderfolgend durch Peptidbindungen verknüpft sind, ausgewählt aus: oder

9. Ein Konstrukt auf Peptid-Basis oder eine Zusammensetzung von 8-15 Aminosäureeinheiten mit einer Sequenz, die eine Teilsequenz der funktionellen Domäne II (Aminosäuren 65-99) von bakterizidem/permeabilitätserhöhendem Protein (BPI) in umgekehrter Reihenfolge ist, wobei das Konstrukt auf Peptid-Basis oder die Zusammensetzung nicht k-l-f-r (naph-a)-q-a-k-(naph-a)-k-g-s-i-k-i ist (SEQ ID NO: 1) ist.

10. Ein derivatisiertes Konstrukt auf Peptid-Basis oder eine Zusammensetzung von 8-15 Aminosäureeinheiten mit einer Sequenz, die eine Teilsequenz einer funktionellen Domäne II (Aminosäuren 65-99) des bakteriziden/permeabilitätserhöhenden Proteins (BPI) in umgekehrter Reihenfolge ist, wobei die Sequenz kovalent an eine hydrophobe Gruppe geknüpft ist, wobei das Konstrukt auf Peptid-Basis oder die Zusammensetzung nicht k-l-f-r (naph-a)-q-a-k(naph-a)-k-g-s-i-k-i ist (SEQ ID NO: 1) ist.

11. Konstrukt oder Zusammensetzung nach Anspruch 10, wobei die hydrophobe Einheit kovalent an den N-Terminus der Sequenz geknüpft ist.

12. Konstrukt oder Zusammensetzung nach Anspruch 10 oder 11, wobei die hydrophobe Einheit R₂ ist, wobei R₂ ein beliebiges ist von: ein zyklisches Molekül mit wenigstens 3 Kohlenstoffatomen, ein heterozyklisches Molekül mit wenigstens 3 Atomen, ein funktionalisiertes zyklisches Molekül mit wenigstens 3 Kohlenstoffatomen oder ein funktionalisiertes heterozyklisches Molekül mit wenigstens 3 Atomen.

13. Konstrukt oder Zusammensetzung nach einem der Ansprüche 10 bis 12, wobei die umgekehrte Untersequenz die Aminosäuren 99-92, 99-91, 99-90, 99-89, 99-88, 99-87, 99-86 oder 99-85 von BPI-Protein ist, und wobei die umgekehrte Untereinheit an den Positionen 95 und 91 substituiert ist.

14. Konstrukt oder Zusammensetzung nach einem der Ansprüche 1 bis 13 mit Heparinneutralisierenden Eigenschaften.

15. Konstrukt oder Zusammensetzung nach einem der Ansprüche 1 bis 13 mit Endothelzellproliferations-inhibierenden Eigenschaften.

16. Konstrukt oder Zusammensetzung nach einem der Ansprüche 1 bis 13 mit antiangiogenen Eigenschaften.

17. Konstrukt oder Zusammensetzung nach einem der Ansprüche 1 bis 16, wobei die Sequenz D-Aminosäureeinheiten enthält.

18. Konstrukt oder Zusammensetzung nach einem der Ansprüche 1 bis 17, wobei die ersten beiden amino-terminalen Aminosäureeinheiten D-Aminosäureeinheiten sind und die letzten beiden carboxy-terminalen Aminosäureeinheiten D-Aminosäureeinheiten sind.

19. Eine pharmazeutische Zusammensetzung, umfassend ein Konstrukt oder eine Zusammensetzung nach einem der Ansprüche 1 bis 18 und ein pharmazeutisch annehmbares Adjuvans, Verdünnungsmittel oder Träger.

20. Konstrukt oder Zusammensetzung nach einem der Ansprüche 1 bis 18 zur Verwendung bei der Therapie.

21. Verwendung eines Konstruktes oder einer Zusammensetzung nach einem der Ansprüche 1 bis 19 zur Herstellung eines Medikaments zum Binden und/oder Neutralisieren einer exogenen oder therapeutisch verabreichten Heparinverbindung, zum Binden und/oder Neutralisieren von Heparin oder zum Behandeln einer mit Heparin in Bezug stehender oder durch Heparin vermittelter Störung, eines Zustands oder einer Krankheit oder zur Herstellung eines Medikaments für eine antimikrobielle Aktivität, zum Binden und/oder Neutralisieren von LPS oder zum Behandeln einer Infektion oder einer Störung, die mit Endotoxin in Bezug steht.

22. Verwendung nach Anspruch 21, wobei das Medikament zur Verabreichung zusammen mit einem anderen therapeutischen Mittel dient.

23. Verwendung nach Anspruch 22, wobei das Medikament zur Verabreichung vor oder nach dem anderen therapeutischen Mittel dient.

24. Verwendung eines Konstruktes oder einer Zusammensetzung nach einem der Ansprüche 1 bis 19 in Kombination mit einem anderen therapeutischen Mittel zur Herstellung eines Medikaments zum Binden und/oder Neutralisieren einer exogenen oder therapeutisch verabreichten Heparinverbindung, zum Binden und/oder Neutralisieren von Heparin oder zum Behandeln einer mit Heparin in Bezug stehender oder durch Heparin vermittelter Störung, eines Zustands oder einer Krankheit oder zur Herstellung eines Medikaments für eine antimikrobielle Aktivität, zum Binden und/oder Neutralisieren von LPS oder zum Behandeln einer Infektion oder einer Störung, die mit Endotoxin in Bezug steht.

25. Verwendung nach einem der Ansprüche 21 bis 24, wobei das Medikament zum Neutralisieren des gerinnungshemmenden Effekts einer exogenen, an ein Säugetier verabreichten Heparinverbindung dient.

26. Verwendung nach Anspruch 25, wobei die Gerinnungszeit des Säugetieres auf einen normalen Zustand gebracht wird.

27. Verwendung nach einem der Ansprüche 21 bis 26, wobei die exogene Heparinverbindung während einer kardiopulmonalen Bypass-Operation, einer Herzkatheterisierung oder einer Angioplastie, Hämodialyse oder an einen Patienten verabreicht worden ist, bei dem das Risiko einer Thrombose, einschließlich einer tiefen Venenthrombose, eines akuten Myocardinfarkts, eines Himschlags oder einer Lungenembolie besteht, oder der daran leidet.

28. Verwendung nach einem der Ansprüche 21 bis 24, wobei das Medikament zum Behandeln einer Störung dient, die mit Endothelzellproliferation assoziiert ist.

29. Verwendung nach einem der Ansprüche 21 bis 24, wobei das Medikament zum Inhibieren der Endothelzellproliferation bei einem Säugetier dient.

30. Verwendung nach einem der Ansprüche 21 bis 24, wobei das Medikament zum Inhibieren von Angiogenese in einem Säugetier dient.

31. Verwendung nach Anspruch 30, wobei die Angiogenese im Auge ist.

32. Verwendung nach Anspruch 31, wobei die Angiogenese im Auge bei der Augen-Neovaskularisierung, proliferativer Retinopathie, retrolentaler Fibroplasie, Makuladegeneration, neovaskulärem Glaukom, oder Diabetes-Augenkrankheit beteiligt ist, einschließlich Diabetes-Iris-Neovaskularisierung oder Retinopathie.

33. Verwendung nach einem der Ansprüche 21 bis 24, wobei das Medikament zum Behandeln eines Säugetieres dient, das an einer Störung, Zustand oder Krankheit leidet, die mit Angiogenese assoziiert ist oder diese beinhaltet.

34. Verwendung nach Anspruch 33, wobei das Medikament zum Inhibieren eines Fortschreitens der Störung, des Zustands oder der Krankheit oder zum Verbessern der Anzeichen und Symptome der Störung, des Zustands oder der Krankheit dient.

35. Verwendung nach Anspruch 33, wobei besagte Störung, die Angiogenese beinhaltet, eine chronische entzündliche Krankheit ist.

36. Verwendung nach Anspruch 35, wobei besagte chronische entzündliche Krankheit chronische Pankreatitis, Dermatose, die mit chronischer Entzündung assoziiert ist, einschließlich Psoriasis, Zirrhose, Asthma, multiple Sklerose, Arthritis, einschließlich rheumatoider Arthritis, reaktiver Arthritis oder chronischer entzündlicher Arthritis, Autoimmunkrankheiten, einschließlich Vaskulitis, Glomerulonephritis, experimenteller allergischer Enzephalomyelitis (EAE), Lupus, Myasthenia gravis, ulzerative Colitis, Crohnsche Krankheit, entzündliche Darmkrankheit, chronische Entzündung, die mit Hämodialyse assoziiert ist, Granulocytentransfusion-assoziiertes Syndrom; Abstoßungsreaktionen nach Allograft- oder Xenograft-Transplantation, einschließlich Transplantat-gegen-Empfänger-Reaktion; oder andere chronische entzündliche Störungen ist.

37. Verwendung nach Anspruch 33, wobei besagte Störung, die Angiogenese beinhaltet, das Wachstum, die Proliferation oder die Metastase von Tumorzellen ist.

38. Verwendung nach Anspruch 37, wobei das Medikament zum Fördern einer Tumor-Regression bei Erwachsenen- oder Kinderonkologie dient, einschließlich dem Reduzieren des Wachstums von festen Tumoren, malignen Wucherungen, lokal fortgeschrittenen Tumoren, metastasierendem Krebs, menschlichen Weichgewebe-Sarcomen, Krebsmetastasen, einschließlich Lymph-Metastasen, malignen Blutzellkrankheiten, Effusionslymphomen (Lymphomen auf Grundlage von Körperhöhlungen), Lungenkrebs einschließlich kleinzelliges Carcinom, Krebsarten, die nicht kleinzelliges Carcinom sind, Brustkrebs, einschließlich kleinzelligem Carcinom oder Ductus-Carcinom, Gastrointestinalkrebse, einschließlich Magenkrebs, Dickdarmkrebs, Colorectalkrebs, Polypen, die mit colorectaler Neoplasie assoziiert sind, Pankreaskrebs, Leberkrebs, urologische Krebse, einschließlich Blasenkrebs, Prostatakrebs, maligne Erkrankungen des weiblichen Genitaltrakts, einschließlich Ovarialcarcinom, Uterus-Endometrium-Krebse oder feste Tumore im Ovarialfollikel, Nierenkrebs, einschließlich Nierencarcinom, Gehirnkrebs, einschließlich intrinsischer Hirntumore, Neuroblastom, Astrocyten-Hirntumore, Gliome, metastatische Tumorzellinvasion im zentralen Nervensystem, Knochenkrebse, einschließlich Osteome, Hautkrebse, einschließlich malignem Melanom, Tumorfortschritt von menschlichen Hautkeratinocyten oder Plattenepithelcarcinom, Hämangiopericytom oder Kaposi-Sarcom.

39. Verwendung nach Anspruch 33, wobei das Medikament zur Behandlung von Arteriosklerose, ischämischer Herzkrankheit, Myocardinfarkt, koronarer Herzkrankheit, Restenose, einschließlich Restenose nach Ballonangiographie, neointimaler Hyperplasie, Zerstörung von interzellulären Verbindungen im Gefäßendothel, Bluthochdruck, Gefäßverletzung, Arterienischämie, Arterienstenose, peripherer Gefäßkrankheit oder Hirnschlag dient.

40. Verwendung nach einem der Ansprüche 21 bis 39, wobei das Medikament zur systemischen oder topischen Verabreichung dient.

41. Verwendung nach Anspruch 40, wobei das Medikament zur oralen, intravenösen, intramuskulären oder subkutanen, intraokularen und retrobulbären, intrathekalen, intraperitonealen, intrapulmonalen oder transdermalen Verabreichung dient.

42. Verwendung nach Anspruch 40, wobei das Medikament zur Verabreichung in der Form von Salben, Augentropfen, Ohrentropfen oder Spülflüssigkeiten dient.

43. Verwendung nach einem der Ansprüche 1 bis 40, wobei das Medikament zur parenteralen Verabreichung in einer Dosis im Bereich von 1 µg/kg bis 100 mg/kg pro Tag dient.

44. In-vitro-Verfahren zum Neutralisieren des gerinnungshemmenden Effekts von Heparin, umfassend das In-Kontakt-Bringen des Heparins mit einem Konstrukt oder Zusammensetzung nach einem der Ansprüche 1 bis 18.

45. Verfahren zum Identifizieren einer derivatisierten Peptidsequenz, die aus der Sequenz abgeleitet ist oder auf dieser beruht, welche aus der Domäne II von bakterizidem/permeabilitäiserhöhendem Protein (BPI) identifiziert und ausgewählt worden ist mit biologischer Aktivität und einer Epithelabsorption von wenigstens 0,001 %, umfassend die Schritte:
(a) Derivatisieren einer Peptidsequenz, basierend auf einer Sequenz, Teilsequenz, umgekehrten Sequenz oder umgekehrten Teilsequenz der Domäne II von BPI, durch kovalente Verknüpfung einer hydrophoben Gruppe oder Gruppen am N-Terminus, C-Terminus oder innerhalb der besagten Peptidsequenz;
(b) Messen der Aktivität besagter derivatisierter Peptidsequenz, die in Schritt (a) erhalten worden ist, wobei die Aktivität eine Eigenschaft oder mehrere Eigenschaften sind von: Heparinbindung, Heparinneutralisierung, Inhibition der Endothelzellproliferation, anti-angiogene Eigenschaft, LPS-Bindung, LPS-Neutralisierung oder antimikrobielle Eigenschaft, und
(c) Messen der Epithelabsorption der besagten derivatisierten Peptidsequenz, die in Schritt (a) erhalten worden ist.

46. Verfahren zum Designen und Identfizieren einer biologisch aktiven Sequenz auf derivatisierter Peptid-Basis, prophylaktisches oder therapeutisches Medikament, das aus der Peptidsequenz abgeleitet ist oder auf ihr basiert, die aus BPI oder einem Fragment davon identifiziert und ausgewählt worden ist, mit einer Epithelabsorption von wenigstens 0,001%, wobei das Verfahren die Schritte umfaßt:
(a) Identifizieren einer Zielpeptidsequenz, die von der Polypeptidsequenz von BPI oder eines Fragments davon abgeleitet ist, oder auf ihr basiert, welche eine in-vitro-Aktivität oder eine in-vivo-Aktivität aufweist, wobei die Aktivität eine oder mehrere Eigenschaften ist von: Heparinbindung, Heparinneutralisierung, Inhibition der Endothelzellproliferation, anti-angiogene Eigenschaft, LPS-Bindung, LPS-Neutralisierung oder antimikrobielle Eigenschaft, und
(b) Konstruieren einer Bibliothek von die Aktivität beibehaltenden Peptidsequenzen minimaler Länge (MinLARPS) durch Substituieren oder Deletieren von Aminosäureeinheiten innerhalb besagter Target-Peptidsequenz;
(c) Messen der Aktivität der besagten MinLARPS, um die minimale Anzahl an Resten zu bestimmen, die notwendig ist, um wenigstens 1% der Aktivität von besagter Zielpolypeptidsequenz beizubehalten, bei der die Aktivität eine oder mehrere Eigenschaften ist von: Heparinbindung, Heparinneutralisierung, Inhibition der Endothelzellproliferation, anti-angiogene Eigenschaft, LPS-Bindung, LPS-Neutralisierung oder antimikrobielle Eigenschaft,
(d) Messen der Epithelabsorption von besagter MinLARPS in in-vitro-Assays, um zu identifizieren, welche der besagten MinLARPS eine Epithelabsorption von wenigstens 0,001 % beibehält;
(e) Synthetisieren von derivatisierten MinLARPS durch chemisches Modifizieren besagter MinLARPS durch kovalente Verknüpfung einer hydrophoben Gruppe oder Gruppen, die an dem N-Terminus, C-Terminus oder innerhalb dieser Sequenz besagter MinLARPS angehängt wird;
(f) Wiederholen der Schritte c) und d) mit besagter derivatisierter MinLARPS.

## Revendications

1. Composition de 8-15 résidus aminoacides liés de manière consécutive par des liaisons peptidiques, ladite composition ayant au moins une ou plusieurs propriétés parmi les propriétés de liaison à l'héparine, de neutralisation de l'héparine, d'inhibition de la prolifération des cellules endothéliales, antiangiogéniques, de liaison au LPS, de neutralisation du LPS ou antimicrobiennes et comprenant une séquence de formule :
KLFR(naph-A)QAR₃,
dans laquelle les symboles peuvent désigner aussi bien un résidu D-aminoacide qu'un résidu L-aminoacide,
dans laquelle R₃ est l'un quelconque parmi K, K(naph-A), K(naph-A)K, K(naph-A)KG, K(naph-A)KGS, K(naph-A)KGSI, K(naph-A)KGSIK ou K(naph-A)KGSIKI ; ou
dans laquelle le groupe carboxy-terminal est amidifié ou non amidifié,
et optionnellement, au moins une substitution conservative de résidus aminoacides ;
dans laquelle la composition n'est pas k-l-f-r(naph-a)-q-a-k-(naph-a)-k-g-s-i-k-i (SEQ ID NO:1).

2. Composition selon la revendication 1 comprenant deux ou plusieurs substitutions conservatives de résidus aminoacides.

3. Composition ou construction à base de peptide, constituée de résidus aminoacides liés de manière consécutive par des liaisons peptidiques choisie parmi :

4. Composition de 8-15 résidus aminoacides liés de manière consécutive par des liaisons peptidiques, ladite composition ayant au moins une propriété parmi les propriétés de liaison à l'héparine, de neutralisation de l'héparine, d'inhibition de la prolifération des cellules endothéliales, antiangiogéniques, de liaison au LPS, de neutralisation du LPS ou antimicrobiennes et comprenant une séquence de formule :
R₁KLFR(naph-A)QAR₃
dans laquelle R₁ est l'un quelconque parmi R₂-CH₂-, R₂-CH₂-CO-, R₂-CO-, R₂-SO_{y}-, ou R₂-PO_{z}- ;
dans laquelle,
y=0-3,
z=1-4 ;
R₂ est un groupement hydrophobe qui est un groupement quelconque parmi une molécule cyclique ayant au moins 3 atomes de carbone, une molécule hétérocyclique ayant au moins 3 atomes, une molécule cyclique fonctionnalisée ayant au moins 3 atomes de carbone, ou une molécule hétérocyclique fonctionnalisée ayant au moins 3 atomes ;
dans laquelle R₃ est l'un quelconque parmi K, K(naph-A), K(naph-A)K, K (naph-A) KG, K(naph-A)KGS, K(naph-A)KGSI, K(naph-A)KGSIK ou K(naph-A)KGSIKI ;
dans laquelle le groupe carboxy-terminal est amidifié ou non amidifié,
et, optionnellement, comprenant au moins une substitution conservative de résidus aminoacides ;
dans laquelle la composition n'est pas k-l-f-r-(naph-a)-q-a-k-(naph-a)-k-g-s-i-k-i (SEQ ID NO:1).

5. Composition selon la revendication 4 comprenant deux ou plusieurs substitutions conservatives de résidus aminoacides.

6. Composition selon la revendication 4, dans laquelle ledit R₂ est un groupement hydrophobe qui est un groupement quelconque parmi (a) un noyau carbocyclique optionnellement substitué, saturé ou partiellement ou complètement insaturé contenant 3 à 8, de préférence 5 ou 6, atomes de carbone ; (b) un noyau hétérocyclique optionnellement substitué, saturé ou partiellement ou complètement insaturé contenant 3 à 8, de préférence 5 ou 6, atomes, dans lequel au moins un atome est un hétéroatome qui est l'un quelconque parmi l'oxygène, l'azote, ou le soufre ; ou (c) un noyau bicyclique optionnellement substitué 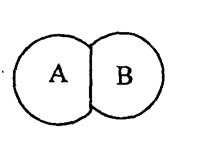 dans lequel les cycles condensés A et B, indépendamment, sont un cycle à 5 ou 6 chaînons, saturé ou partiellement ou complètement insaturé, et comprennent des atomes de carbone et optionnellement un à trois hétéroatomes choisis parmi l'oxygène, le soufre, ou l'azote ; dans lequel s'il y a plus d'un hétéroatome, chacun pouvant être identique ou différent.

7. Composition selon la revendication 4, dans laquelle ledit R₂ est un groupement hydrophobe qui est un groupement quelconque parmi les suivants : biotine, 2-biphénylène, 2-anthraquinone, 2-benzofurane, 2-indole, 1-isoquinoléine, hydroxyphényle, 2-quinoléine, 1-[3-(3,4-dihydroxycinnamoyl)-1,3,4,5-tétrahydroxycyclohexyle], 1-(3,5-dichloro-2-hydroxyphényle), 1-(3,5-diiodo-2-hydroxyphényle), 1-(3,5-dinitro-2-hydroxyphényle), 1-(4-azido-2-hydroxyphényle), 4-biphényle, 2-biphényle, 1-naphtyle, 2-naphtyle, 3-amino-2-naphtyle, 3-chloro-2-nitrophényle, 3,4-dihydroxy-phényle, 3,4,5-trihydroxyphényle, 2-chloro-3-nitrophényle, 5-azido-2-nitrophényle, 3-amino-2-pyrazyle, 2-benzyloxycarbonyl-éthyle, 2-thiényle, 2-(3,4-dihydroxyphényl)éthylène, 5-bromo-3-indoleméthylène, 2-(4-hydroxy-3-méthoxyphényl)éthylène, 2-(3-chlorophényl)éthylène, 2-pyrazyle, 4-imidazolyle, 2-imino-1-imidazolidyle, pyridyle, 3-pipéridyle, 4-pipéridyle, fluorescéine, 2-(4-amino-3,5,6-trichloropyridyle), 3-(2-chloro-6-fluorophényl)-5-méthylisoxazolyle, ou 4-azido-phényle.

8. Composition ou construction à base de peptide, constituée de résidus aminoacides liés de manière consécutive par des liaisons peptidiques choisie parmi :
2-biphénylcarbonyl-k-l-f-r-(naph-a)-q-a-k (SEQ ID NO:19) ;
4-biphénylcarbonyl-k-l-f-r-(naph-a)-q-a-k (SEQ ID NO:20) ;
2-naphtylacétyl-k-l-f-r-(naph-a)-q-a-k (SEQ ID NO:21) ;
1-naphtylacétyl-k-l-f-r-(naph-a)-q-a-k (SEQ ID NO:22) ;
2-biphénylènecarbonyl-k-l-f-r-(naph-a)-q-a-k (SEQ ID NO:23) ;
4-biphénylcarbonyl-k-l-f-k-(naph-a)-q-a-k (SEQ ID NO:24) ;
4-biphénylcarbonyl-k-l-f-r-(biphényl-a)-q-a-k (SEQ ID NO:25) ; ou
4-biphénylcarbonyl-k-l-f-r-(biphényl-a)-q-a-k (SEQ ID NO:26).

9. Composition ou construction à base de peptide, ayant une longueur de 8-15 résidus aminoacides ayant une séquence qui est une sous-séquence du domaine II fonctionnel (aminoacides 65-99) de la protéine BPI ("bactericidal/permeability increasing (BPI) protein") dans l'ordre inverse, dans laquelle la composition ou construction à base de peptide n'est pas k-l-f-r-(naph-a)-q-a-k-(naph-a)-k-g-s-i-k-i (SEQ ID NO:1).

10. Composition ou construction à base de peptide dérivatisée ayant une longueur de 8-15 résidus aminoacides et ayant une séquence qui est une sous-séquence du domaine II fonctionnel (aminoacides 65-99) de la protéine BPI ("bactericidal/permeability increasing (BPI) protein") dans l'ordre inverse, ladite séquence étant liée de manière covalente à un groupement hydrophobe, dans laquelle la composition ou construction à base de peptide n'est pas k-l-f-r-(naph-a)-q-a-k-(naph-a)-k-g-s-i-k-i (SEQ ID NO:1).

11. Composition ou construction selon la revendication 10, dans laquelle le groupement hydrophobe est lié de manière covalente à l'extrémité N-terminale de la séquence.

12. Composition ou construction selon la revendication 10 ou 11, dans laquelle le groupement hydrophobe est R₂, où R₂ est un groupement quelconque parmi une molécule cyclique ayant au moins 3 atomes de carbone, une molécule hétérocyclique ayant au moins 3 atomes, une molécule cyclique fonctionnalisée ayant au moins 3 atomes de carbone, ou une molécule hétérocyclique fonctionnalisée ayant au moins 3 atomes.

13. Composition ou construction selon l'une quelconque des revendications 10 à 12, dans laquelle la sous-séquence inverse consiste en les aminoacides 99-92, 99-91, 99-90, 99-89, 99-88, 99-87, 99-86, ou 99-85 de la protéine BPI, et dans laquelle la sous-séquence inverse est substituée aux positions 95 et 91.

14. Composition ou construction selon l'une quelconque des revendications 1 à 13 ayant des propriétés de neutralisation de l'héparine.

15. Composition ou construction selon l'une quelconque des revendications 1 à 13 ayant des propriétés d'inhibition de la prolifération des cellules endothéliales.

16. Composition ou construction selon l'une quelconque des revendications 1 à 13 ayant des propriétés antiangiogéniques.

17. Composition ou construction selon l'une quelconque des revendications 1 à 16, dans laquelle la séquence contient des résidus D-aminoacides.

18. Composition ou construction selon l'une quelconque des revendications 1 à 17, dans laquelle les deux premiers résidus aminoacides amino-terminaux sont des résidus D-aminoacides et les deux derniers résidus aminoacides carboxy-terminaux sont des résidus D-aminoacides.

19. Composition pharmaceutique comprenant une composition ou construction selon l'une quelconque des revendications 1 à 18 et un adjuvant, diluant, ou support, pharmaceutiquement acceptable.

20. Composition ou construction selon l'une quelconque des revendications 1 à 18 destinée à être utilisée en thérapeutique.

21. Utilisation d'une composition ou construction selon l'une quelconque des revendications 1 à 19 pour la fabrication d'un médicament destiné à lier et/ou à neutraliser un composé héparinique exogène ou administré thérapeutiquement, à lier et/ou à neutraliser l'héparine, ou à traiter un trouble, une affection ou une maladie lié(e) à l'héparine ou médié(e) par l'héparine, ou pour la fabrication d'un médicament destiné à une activité antimicrobienne, à la liaison et/ou à la neutralisation du LPS, ou au traitement d'une infection ou d'un trouble associé(e) à une endotoxine.

22. Utilisation selon la revendication 21, dans laquelle le médicament est destiné à une administration concomitante avec un autre agent thérapeutique.

23. Utilisation selon la revendication 22, dans laquelle le médicament est destiné à une administration avant ou après l'autre agent thérapeutique.

24. Utilisation d'une composition ou construction selon l'une quelconque des revendications 1 à 19 en combinaison avec un autre agent thérapeutique pour la fabrication d'un médicament destiné à lier et/ou à neutraliser un composé héparinique exogène ou administré thérapeutiquement, à lier et/ou à neutraliser l'héparine, ou à traiter un trouble, une affection ou une maladie lié(e) à l'héparine ou médié(e) par l'héparine, ou pour la fabrication d'un médicament destiné à une activité antimicrobienne, à la liaison et/ou à la neutralisation du LPS, ou au traitement d'une infection ou d'un trouble associé(e) à une endotoxine.

25. Utilisation selon l'une quelconque des revendications 21 à 24, dans laquelle le médicament est destiné à neutraliser l'effet anticoagulant d'un composé héparinique exogène administré à un mammifère.

26. Utilisation selon la revendication 25, dans laquelle le temps de coagulation dudit mammifère est revenu à la normale.

27. Utilisation selon l'une quelconque des revendications 21 à 26, dans laquelle le composé héparinique exogène a été administré pendant une opération chirurgicale avec circulation extracorporelle, un cathétérisme cardiaque ou une angioplastie, une hémodialyse, ou à un patient exposé au risque de ou souffrant de thrombose, y compris une thrombose veineuse profonde, d'un infarctus aigu du myocarde, d'un accident cérébrovasculaire ou d'une embolie pulmonaire.

28. Utilisation selon l'une quelconque des revendications 21 à 24, dans laquelle le médicament est destiné à traiter un trouble associé à une prolifération des cellules endothéliales.

29. Utilisation selon l'une quelconque des revendications 21 à 24, dans laquelle le médicament est destiné à inhiber la prolifération des cellules endothéliales chez un mammifère.

30. Utilisation selon l'une quelconque des revendications 21 à 24, dans laquelle le médicament est destiné à inhiber une angiogenèse chez un mammifère.

31. Utilisation selon la revendication 30, dans laquelle ladite angiogenèse est dans l'oeil.

32. Utilisation selon la revendication 31, dans laquelle l'angiogenèse dans l'oeil est impliquée dans une néovascularisation oculaire, une rétinopathie proliférante, une fibroplasie rétrocristallinienne, une dégénérescence maculaire, un glaucome néovasculaire ou une maladie oculaire diabétique, notamment une rétinopathie ou néovascularisation de l'iris diabétique.

33. Utilisation selon l'une quelconque des revendications 21 à 24, dans laquelle le médicament est destiné à traiter un mammifère souffrant d'un trouble, d'une affection ou d'une maladie associé(e) à ou impliquant une angiogenèse.

34. Utilisation selon la revendication 33, dans laquelle le médicament est destiné à inhiber la progression du trouble, de l'affection ou de la maladie, ou à améliorer les signes et les symptômes du trouble, de l'affection ou de la maladie.

35. Utilisation selon la revendication 33, dans laquelle ledit trouble impliquant une angiogenèse est une maladie inflammatoire chronique.

36. Utilisation selon la revendication 35, dans laquelle ladite maladie inflammatoire chronique est la pancréatite chronique, la dermatose associée à une inflammation chronique,. notamment le psoriasis, la cirrhose, l'asthme, la sclérose en plaques, l'arthrite, notamment la polyarthrite rhumatoïde, l'arthrite réactive ou l'arthrite inflammatoire chronique, des maladies auto-immunes, notamment la vascularite, la glomérulonéphrite, l'encéphalomyélite allergique expérimentale (EAE), le lupus, la myasthénie gravis, la rectocolite hémorragique, la maladie de Crohn, les maladies inflammatoires intestinales, l'inflammation chronique associée à l'hémodialyse, le syndrome associé à la transfusion de granulocytes ; les réactions de rejet après une allogreffe ou une xénogreffe, notamment la réaction du greffon contre l'hôte ; ou d'autres troubles inflammatoires chroniques.

37. Utilisation selon la revendication 33, dans laquelle ledit trouble impliquant une angiogenèse est la croissance, la prolifération ou la métastase de cellules tumorales.

38. Utilisation selon la revendication 37, dans laquelle le médicament est destiné à favoriser la régression des tumeurs en oncologie adulte ou pédiatrique, notamment à réduire la croissance des malignités/tumeurs solides, des tumeurs localement avancées, du cancer métastasique, des sarcomes des tissus mous humains, des métastases cancéreuses, y compris des métastases lymphatiques, des malignités des cellules sanguines, des lymphomes à épanchement (lymphomes basés dans des cavités corporelles), du cancer du poumon, y compris du carcinome à petites cellules, des cancers "non à petites cellules", du cancer du sein, y compris du carcinome à petites cellules ou du carcinonne canalaire, des cancers gastrointestinaux, y compris du cancer de l'estomac, du cancer du côlon, du cancer colorectal, des polypes associés à une néoplasie colorectale, du cancer du pancréas, du cancer du foie, des cancers urologiques, y compris du cancer de la vessie, du cancer de la prostate, des malignités de l'appareil génital féminin, y compris du cancer de l'ovaire, des cancers endométriaux utérins, ou des tumeurs solides dans le follicule ovarien, du cancer du rein, y compris du carcinome des cellules rénales, du cancer du cerveau, y compris des tumeurs cérébrales intrinsèques, du neuroblastome, des tumeurs cérébrales astrocytaires, des gliomes, de l'invasion des cellules tumorales métastasiques dans le système nerveux central, des cancers des os, y compris des ostéomes, des cancers de la peau, y compris du mélanome malin, de l'évolution tumorale des kératinocytes cutanés humains, ou du cancer spinocellulaire, de l'hémangiopéricytome, ou du sarcome de Kaposi.

39. Utilisation selon la revendication 33, dans laquelle le médicament est destiné au traitement de l'athérosclérose, de la cardiopathie ischémique, de l'infarctus du myocarde, de la coronaropathie, de la resténose, y compris la resténose faisant suite à une angiographie par ballonnet, de l'hyperplasie néointimale, de la dissociation des jonctions intercellulaires dans l'endothélium vasculaire, de l'hypertension, des lésions vasculaires, de l'ischémie artérielle, de la sténose artérielle, des maladies vasculaires périphériques ou de l'accident cérébrovasculaire.

40. Utilisation selon l'une quelconque des revendications 21 à 39 , dans laquelle le médicament est destiné à une administration systémique ou topique.

41. Utilisation selon la revendication 40, dans laquelle le médicament est destiné à une administration orale, intraveineuse, intramusculaire ou sous-cutanée, intraoculaire et rétrobulbaire, intrathécale, intrapéritonéale, intrapulmonaire, ou transdermique.

42. Utilisation selon la revendication 40, dans laquelle le médicament est destiné à une administration sous forme de pommades, de gouttes ophtalmiques, de gouttes auriculaires, ou de fluides pour irrigation.

43. Utilisation selon l'une quelconque des revendications 1 à 40, dans laquelle le médicament est destiné à une administration parentérale à une dose allant de 1 µg/kg à 100 mg/kg par jour.

44. Procédé in vitro pour neutraliser l'effet anticoagulant de l'héparine comprenant la mise en contact de l'héparine avec une composition ou construction selon l'une quelconque des revendications 1 à 18.

45. Procédé pour identifier une séquence peptidique dérivatisée dérivée de ou basée sur la séquence identifiée et choisie dans le domaine II de la protéine BPI ("bactericidal/permeability increasing (BPI) protein") ayant une activité biologique et une absorption épithéliale d'au moins 0,001 % comprenant les étapes de :
(a) dérivatisation d'une séquence peptidique basée sur une séquence, sous-séquence, séquence inverse ou sous-séquence inverse du domaine II de BPI par liaison covalente d'un ou plusieurs groupements hydrophobes à l'extrémité N-terminale, C-terminale ou à l'intérieur de ladite séquence peptidique ;
(b) mesure de l'activité de ladite séquence peptidique dérivatisée obtenue dans l'étape (a) où l'activité est une ou.plusieurs propriétés quelconques parmi les propriétés de liaison à l'héparine, de neutralisation de l'héparine, d'inhibition de la prolifération des cellules endothéliales, antiangiogéniques, de liaison au LPS, de neutralisation du LPS ou antimicrobiennes et
(c) mesure de l'absorption épithéliale de ladite séquence peptidique dérivatisée obtenue dans l'étape (a).

46. Procédé pour concevoir et identifier une séquence à base de peptide dérivatisée biologiquement active, un médicament prophylactique ou thérapeutique dérivé de ou basé sur la séquence peptidique identifiée et choisie parmi BPI ou un de ses fragments avec une absorption épithéliale d'au moins 0,001 %, ledit procédé comprenant les étapes de :
(a) identification d'une séquence peptidique-cible dérivée de ou basée sur la séquence polypeptidique de BPI ou d'un de ses fragments qui présente une activité *in vitro* ou *in vivo* où l'activité est une ou plusieurs propriétés quelconques parmi les propriétés de liaison à l'héparine, de neutralisation de l'héparine, d'inhibition de la prolifération des cellules endothéliales, antiangiogéniques, de liaison au LPS, de neutralisation du LPS ou antimicrobiennes ;
(b) construction d'une banque de séquences peptidiques conservant l'activité, d'une longueur minimale, (MinLARPS) par substitution ou délétion de résidus aminoacides dans ladite séquence peptidique-cible ;
(c) mesure de l'activité desdites MinLARPS pour déterminer le nombre minimal de résidus nécessaires pour conserver une activité égale au moins à 1 % de celle de ladite séquence polypeptidique-cible où l'activité est une ou plusieurs propriétés quelconques parmi les propriétés de liaison à l'héparine, de neutralisation de l'héparine, d'inhibition de la prolifération des cellules endothéliales, antiangiogéniques, de liaison au LPS, de neutralisation du LPS ou antimicrobiennes ;
(d) mesure de l'absorption épithéliale desdites MinLARPS dans des tests *in vitro* pour identifier lesquelles desdites MinLARPS conservent une absorption épithéliale d'au moins 0,001 % ;
(e) synthèse de MinLARPS dérivatisées par modification chimique desdites MinLARPS par liaison covalente d'un ou plusieurs groupements hydrophobes liés à l'extrémité N-terminale, C-terminale ou à l'intérieur de la séquence desdites MinLARPS ;
(f) répétition des étapes (c) et (d) avec lesdites MinLARPS dérivatisées.
